# EUROPÄISCHE PATENTSCHRIFT

(11) **EP 2 804 595 B1**
(45) Veröffentlichungstag und Bekanntmachung des Hinweises auf die Patenterteilung: **16.09.2015**
(21) Anmeldenummer: 13700054.3
(22) Anmeldetag: 04.01.2013
(51) Int. Cl.: A61K 9/48, A61K 31/015, A61K 31/045, A61K 31/125, A61K 31/35, A61K 45/06, A61P 1/16, A61P 13/00, A61P 17/00, A61P 29/00, A61P 37/00, A61P 31/16, A61P 11/08, A61P 11/06

(54) **SYSTEMISCHE DARREICHUNGSFORMEN MIT KONTROLLIERTER FREISETZUNG UND VERBESSERTER STABILITÄT**
SYSTEMIC ADMINISTRATION FORMS WITH CONTROLLED RELEASE AND IMPROVED STABILITY
FORMES GALÉNIQUES SYSTÉMIQUES À LIBÉRATION CONTROLÉE ET À STABILITÉ AMÉLIORÉE

(30) Priorität: 20.01.2012 DE 102012000982; 31.01.2012 DE 102012001731
(43) Veröffentlichungstag der Anmeldung: 26.11.2014
(73) Patentinhaber: Maria Clementine Martin Klosterfrau Vertriebsgesellschaft mbH, 50670 Köln (DE)
(72) Erfinder: MIETHING, Holger, 12107 Berlin (DE); GREVE, Harald, 40545 Düsseldorf (DE)
(74) Vertreter: Von Rohr Patentanwälte Partnerschaft mbB
(86) Internationale Anmeldenummer: PCT/EP2013/000010
(87) Internationale Veröffentlichungsnummer: WO 2013/107609

(56) Entgegenhaltungen:
- WO-A2-94/28895
- US-A1- 2009 098 275
- DATABASE CA [Online] CHEMICAL ABSTRACTS SERVICE, COLUMBUS, OHIO, US; 22. Dezember 2011 (2011-12-22), CHEN, SHIZHONG ET AL: "Preparation of refined Magnolia biondii oil soft capsule for treating allergic rhinitis and related disease", XP002694131, gefunden im STN Database accession no. 156:21694 -& CN 102 258 573 A (PEOP. REP. CHINA) 30. November 2011 (2011-11-30)

## Beschreibung

Die vorliegende Erfindung betrifft das technische Gebiet systemischer Darreichungsformen, insbesondere systemischer Darreichungsformen mit kontrollierter Freisetzung der Wirksubstanzen.

Insbesondere betrifft die vorliegende Erfindung eine monoterpenhaltige Darreichungsform für die perorale Applikation in Form einer Kapsel, insbesondere in Form einer Kapsel mit Kern/Hülle-Struktur mit mehrschichtiger Hülle.

Des Weiteren betrifft die Erfindung ein Verfahren zur Herstellung der erfindungsgemäßen Darreichungsform sowie deren Verwendung, insbesondere bei medizinischen Applikationen und als Arzneimittel.

Bei Terpenen handelt es sich im Allgemeinen um Naturstoffe, welche als Bestandteil der sogenannten ätherischen Öle in Form von Flüssigkeiten aus Pflanzen oder deren Bestandteilen isoliert werden können. Sie sind oftmals Duft- und Geschmacksstoffe, welche im Bereich der Lebensmittelindustrie oder der Kosmetikindustrie Anwendung finden. Daneben gewinnt jedoch auch der Einsatz von Terpenen für medizinische Zwecke an Bedeutung, da sich für eine Vielzahl von Terpenen pharmakologische Wirkungen nachweisen lassen.

Terpene stellen formal Polymerisationsprodukte des Isoprens dar, wobei nach der Anzahl der Isoprenreste zwischen Monoterpenen (C₁₀-Einheiten), Sesquiterpenen (C₁₅-Einheiten), Diterpenoiden (C₂₀-Einheiten), Sesterterpenen (C₂₅-Einheiten), Triterpenen (C₃₀-Einheiten), Tetraterpenen (C₄₀-Einheiten) und Polyterpenen unterschieden wird (vgl. RÖMPP-Chemielexikon, 10. Auflage, Georg Thieme Verlag, Stuttgart/New York, 1999, Seiten 4449 und 4450, Stichwort "Terpen(oid)e").

Darüber hinaus können Terpene auch als pharmakologische Wirksubstanzen Ausgangsstoffe für die Herstellung von Arzneimitteln oder Vitaminpräparaten sowie aufgrund ihrer oftmals bakterioziden bzw. pestiziden Wirkungen in der Landwirtschaft eingesetzt werden.

Speziell für eine Anzahl von Monoterpenen sind pharmakologische Wirkungen in der Bekämpfung von Krankheiten bei erfolgter systemischen Behandlungen nachgewiesen, wobei insbesondere Menthol und 1,8-Cineol zu nennen sind.

1,8-Cineol gehört zu den bicyclischen Epoxy-Monoterpenen, genauer gesagt den Limonenoxiden. Synonyme Bezeichnungen für 1,8-Cineol mit der chemischen Summenformel C₁₀H₁₈O sind Eucalyptol, Limonen-1,8-oxid, 1,8-Epoxy-p-menthan oder 1,3,3-Trimethyl-2-oxabicyclo[2.2.2]octan. Es handelt sich um eine farblose Flüssigkeit mit würzigem, campherähnlichem Geruch mit einem Schmelzpunkt von +1,5 °C und einem Siedepunkt von 176 bis 177 °C, welche in Wasser unlöslich, aber mit den meisten organischen Lösemitteln mischbar ist.

Natürlich kommt 1,8-Cineol als Hauptbestandteil des Eukalyptusöls (Eukalyptusöl enthält bis zu 85 Gew.-% 1,8-Cineol), aber auch in anderen Pflanzen vor, so z. B. in Minze, Heilsalbei, Thymian, Basilikum und im Teebaum. Darüber hinaus ist 1,8-Cineol beispielsweise in Niaouli-, Juniperus-, Piper-, Cannabis-, Kajeput-, Salbeiöl, Myrtenöl und anderen ätherischen Ölen enthalten.

Technisches 1,8-Cineol, welches im Allgemeinen 99,6- bis 99,8-%ig ist, wird im Allgemeinen durch fraktionierte Destillation von Eukalyptusöl gewonnen.

1,8-Cineol wird insbesondere als Expektorans bei Bronchialkatarrhen und anderen Atemwegserkrankungen vorwiegend in der Veterinärmedizin, aber darüber hinaus auch als Aromastoff in der Parfümindustrie angewendet. Darüber hinaus wird 1,8-Cineol in der Zahnmedizin bei der Revision von Wurzelfüllungen verwendet.

In physiologischer Hinsicht wirkt 1,8-Cineol in den oberen und unteren Atemwegen, insbesondere in der Lunge und den Nebenhöhlen, schleimlösend und bakterizid. Außerdem hemmt es bestimmte Neurotransmitter, welche für die Verengung der Bronchien verantwortlich sind. Bei chronisch-obstruktiven Lungenerkrankungen und Asthma bronchiale kann durch Gabe von reinem 1,8-Cineol die Lungenfunktion verbessert werden. Aufgrund seiner kortikosteroidartigen Wirkung wird es im Fall schwerwiegender Atemwegserkrankungen als Substitut zu oder in Komedikation mit Kortikosteroiden unter systemischer Applikation angewendet. 1,8-Cineol kann grundsätzlich sowohl topisch, z. B. inhalativ, oder aber systemisch, insbesondere in Form von Kapseln, angewendet werden.

Als Wirkstoff besitzt 1,8-Cineol also schleimlösende wie entzündungshemmende Wirkungen. Bei systemischer Anwendung wird 1,8-Cineol leicht resorbiert und gelangt über die Blutbahn in den Atmungsorganen zur Wirkung. 1,8-Cineol kann auf diese Weise beispielsweise entzündliche Sekrete sowie zähen Schleim in den Luftwegen verflüssigen und entzündlichen Prozessen in den Atemwegen entgegenwirken. Ein Sekretstau wird so verhindert, das Abhusten erleichtert, die Funktion der für die Reinigung zuständigen Flimmerhärchen in den Bronchien und der Nase unterstützt und somit die Durchlüftung der Atemwege verbessert. Im Bereich der oberen Luftwege schwinden die Behinderung der Nasenatmung bei Schnupfen und die Benommenheit des Kopfes.

Für weitergehende Einzelheiten zu dem Wirkstoff 1,8-Cineol kann beispielsweise verwiesen werden auf RÖMPP Chemielexikon, Georg Thieme Verlag, Stuttgart/New York, 10. Auflage, Band 1, 1996, Seite 752, Stichwort: "Cineol", sowie die dort referierte Literatur.

In den zu derselben Patentfamilie gehörenden Druckschriften DE 43 19 554 C2, DE 43 19 556 C2 und WO 94/28895 A2 wird eine Kombinationstherapie mit peroral verabreichten Terpenverbindungen, insbesondere 1,8-Cineol oder Menthol, einerseits und ebenfalls systemisch, insbesondere peroral verabreichten Kortikosteroiden andererseits zur entzündungshemmenden Behandlung von systemisch steroidpflichtigem chronischem Asthma bronchiale beschrieben, wobei der Einsatz der peroral verabreichten Terpenverbindungen im Rahmen einer Dauertherapie den Bedarf systemischer Kortikosteroide reduzieren soll. Zum Einsatz kommen bevorzugt magensaftresistente, aber dünndarmlösliche Kapseln mit dem terpenbasierten Wirkstoff.

Für die systemische Anwendung von 1,8-Cineol und anderen Terpenen sind im Handel verschiedene Präparate, insbesondere auf Basis von im Allgemeinen magensaftresistenten, aber dünndarmlöslichen Kapseln, verfügbar.

Die systemische Behandlung mit Terpenen, insbesondere mit Monoterpenen, gestaltet sich jedoch aufwendig und kompliziert, da es sich bei Terpenen oftmals um reaktive Verbindungen handelt, die unter Lichteinwirkung bzw. in Gegenwart von Sauerstoff reagieren und ihre positiven pharmakologischen Eigenschaften verlieren. Darüber hinaus kann es aufgrund der Reaktivität der Monoterpene auch zu einer unerwünschten Wechselwirkung mit dem Wandmaterial der Kapseln kommen, in welchen die Monoterpene eingeschlossen sind. Weiterhin ist problematisch, dass die Terpene oftmals sehr intensive Riech- und Duftstoffe sind, welche nicht in unverdünnter Form angewandt werden können, wodurch eine genaue Dosierung erschwert wird.

Mit den meisten der im Handel verfügbaren Kapseln mit monoterpenhaltigen Wirksubstanzen, insbesondere mit 1,8-Cineol als Wirksubstanz, ist im Allgemeinen nicht immer eine optimale Langzeitstabilisierung oder ein optimaler Schutz vor dem Umgebungsmilieu, insbesondere gegenüber Luftoxidation, gegeben. Eine solche Langzeitstabilisierung kann zwar durch die Verwendung von Mikrokapseln in sogenannten Kapsel-in-Kapsel-Systemen erreicht werden; die Herstellung derartiger Kapsel-in-Kapsel-Systeme ist jedoch äußerst aufwendig und kostspielig und kann nur von wenigen spezialisierten Anbietern durchgeführt werden.

Die systemische Anwendung von Monoterpenen wird durch die Reaktivität der Monoterpene auch in der Hinsicht erschwert, als diese das aggressive, stark saure Milieu des Magens unbeschadet überstehen müssen, um im Dünndarm resorbiert zu werden und anschließend eine systemische Wirkung auszulösen. Aus diesem Grund werden oftmals Dosierformen, insbesondere Kapseln, welche mit einer magensaftresistenten, aber dünndarmlöslichen Beschichtung, einer sogenannten Retard-Beschichtung, versehen sind, eingesetzt. Als Retard-Beschichtungen kommen insbesondere Lacksysteme, wie beispielsweise Schellack, oder synthetische Polymere, wie beispielsweise (Meth-)Acrylsäure bzw. (Meth-)Acrylaten, zum Einsatz.

Die im Stand der Technik eingesetzten Retard-Beschichtungssysteme erfordern jedoch oftmals den Einsatz von unerwünschten Weichmachern, wie beispielsweise Phthalaten, und führen zu mechanisch nur gering belastbaren und optisch nicht immer ansprechenden Beschichtungen. Insbesondere die derzeit üblicherweise verwendeten Beschichtungen auf Basis von (Meth-)Acrylsäuren bzw. (Meth-)Acrylaten neigen dazu, sich von der Kapsel abzulösen, was sich in opaken Flecken auf der Kapseloberfläche äußert. Die Eintrübung der Kapseloberfläche beeinflusst zwar in der Regel nicht die Verwendbarkeit der Kapseln, führt jedoch oftmals zu Beanstandungen seitens des Handels und der Endverbraucher, welche der Auffassung sind, ein mangelbehaftetes Produkt erworben zu haben.

Darüber hinaus ist die Erzeugung einer Retard-Beschichtung mit herkömmlichen Beschichtungsverfahren und Einsatzstoffen nur unter hohem apparativen Aufwand zu bewerkstelligen und mit einer hohen Fehlerquote behaftet, so dass ein Teil der produzierten Kapseln nicht in den Handel gelangt, sondern vielmehr aussortiert wird. Insbesondere müssen Beschichtungen auf Basis von (Meth-)Acrylaten bzw. (Meth-)Acrylsäure bei erhöhten Temperaturen (z. B. 80 °C) verarbeitet werden, was apparativ aufwendig ist (ebenso wie die Homogenisierung und ständige Durchmischung vor dem Aufbringen). Infolgedessen sind Durchsatzraten bei der Herstellung relativ gering.

Des Weiteren erfolgt mit den aus dem Stand der Technik bekannten Kapsel-systemen, insbesondere zur Verkapselung von Monoterpenen, speziell von 1,8-Cineol, eine kontrollierte oder retardierte bzw. verzögerte Freisetzung oftmals nicht stets optimiert bzw. ist bisweilen nicht immer möglich. Ebenfalls ist bei den aus dem Stand der Technik bekannten Wirkstoffkapseln oftmals auch nicht immer eine optimale Dosierung des Wirkstoffs möglich. Darüber hinaus gelingt mit den aus dem Stand der Technik bekannten Kapselsystemen oftmals keine optimale Geschmacks- und/oder Geruchsmaskierung.

So betrifft die CN 102258573 A Weichkapseln für die orale Applikation, welche 1 bis 90 Vol.-% Öl der Pflanze *Magnolia biondii,* 1 bis 90 Vol.-% einer öligen Phase, 0 bis 50 Vol.-% eines Emulgators sowie 0 bis 3 Vol.-% eines Lösemittels enthalten.

Weiterhin betrifft die US 2009/0098275 A1 pharmazeutische Zusammensetzungen in Form von Tabletten, Kapseln oder Pellets, insbesondere Weichkapseln, mit magensaftresistenter Beschichtung, wobei die magensaftresistente Beschichtung auf einer Kombination von ethylcellulosehaltigen Substanzen sowie Alginat bzw. Alginsäuren basiert.

Daher liegt der vorliegenden Erfindung die Aufgabe zugrunde, eine effiziente, für die perorale Applikation bestimmte Darreichungsform für monoterpenhaltige Wirksubstanzen, insbesondere für 1,8-Cineol, und ein entsprechendes Herstellungsverfahren für diese Darreichungsformen bereitzustellen, wodurch die zuvor geschilderten, im Zusammenhang mit dem Stand der Technik auftretenden Nachteile und Nebenwirkungen wenigstens teilweise vermieden oder aber wenigstens zumindest teilweise abgeschwächt werden sollen.

Insbesondere liegt der vorliegenden Erfindung die Aufgabe zugrunde, eine effiziente, für die perorale Applikation bestimmte Darreichungsform für monoterpenhaltige Wirksubstanzen bereitzustellen, welche ein verbessertes Freisetzungsprofil und/oder eine verbesserte Langzeitstabilisierung in Bezug auf die Wirksubstanz ermöglicht. Insbesondere soll dabei auch der Schutz der Wirksubstanz vor dem Umgebungsmilieu, insbesondere gegen Oxidation mit Luftsauerstoff, verbessert werden und zudem eine verbesserte Dosierung und/oder Handhabung des Wirkstoffs, insbesondere auch im Rahmen des Herstellungsverfahrens für diese Darreichungsformen, gewährleistet werden.

Des Weiteren liegt der vorliegenden Erfindung die Aufgabe zugrunde, ein vereinfachtes Verfahren zur Herstellung einer effizienten, für die perorale Applikation bestimmte Darreichungsform für monoterpenhaltige Wirksubstanzen bereitzustellen, welche gegenüber den bisherigen Verfahren apparativ weniger aufwendig ist und kostengünstiger bzw. effizienter durchzuführen ist.

Zur Lösung des zuvor geschilderten Problems schlägt die vorliegende Erfindung - gemäß einem ersten Erfindungsaspekt - eine monoterpenhaltige Darreichungsform für die perorale Applikation in Form einer Kapsel gemäß Anspruch 1 vor; weitere, insbesondere bevorzugte und/oder vorteilhafte Ausgestaltungen dieses Erfindungsaspekts sind Gegenstand der diesbezüglichen Unteransprüche.

Weiterer Gegenstand der vorliegenden Erfindung - gemäß einem zweiten Erfindungsaspekt - ist ein Verfahren zur Herstellung einer monoterpenhaltigen Darreichungsform für die perorale Applikation in Form einer Kapsel nach Anspruch 11; weitere, insbesondere bevorzugte und/oder vorteilhafte Ausgestaltungen dieses Erfindungsaspekts sind Gegenstand des diesbezüglichen Unteranspruchs.

Schließlich ist weiterer Gegenstand der vorliegenden Erfindung - gemäß einem dritten Erfindungsaspekt - ein Arzneimittel oder Medizinprodukt gemäß Anspruch 10.

Es versteht sich von selbst, dass Ausgestaltungen, Ausführungsformen, Vorteile oder dergleichen, welche nachfolgend - zu Zwecken der Vermeidung von unnötigen Wiederholungen - nur zu einem Erfindungsaspekt ausgeführt werden, selbstverständlich auch in Bezug auf die übrigen Erfindungsaspekte entsprechend gelten.

Weiterhin versteht es sich von selbst, dass bei nachfolgenden Angaben von Werten, Zahlen und Bereichen die diesbezüglichen Werte-, Zahlen- und Bereichsangaben nicht beschränkend zu verstehen sind; es versteht sich für den Fachmann von selbst, dass einzelfallbedingt oder anwendungsbezogen von dem angegebenen Bereich bzw. Angaben abgewichen werden kann, ohne dass der Rahmen der vorliegenden Erfindung verlassen ist.

Zudem gilt, dass alle im Folgenden genannten Werte- bzw. Parameterangaben oder dergleichen grundsätzlich mit genormten bzw. standardisierten oder explizit angegebenen Bestimmungsverfahren oder aber mit dem Fachmann auf diesem Gebiet an sich geläufigen Bestimmungsmethoden ermittelt bzw. bestimmt werden können.

Ferner ist bei allen nachstehend genannten relativen bzw. prozentualen, insbesondere gewichtsbezogenen Mengenangaben zu beachten, dass diese Angaben im Rahmen der erfindungsgemäßen Zusammensetzung vom Fachmann derart auszuwählen bzw. zu kombinieren sind, dass in der Summe - gegebenenfalls unter Einbeziehung weiterer Komponenten bzw. Inhaltsstoffe bzw. Zusatzstoffe bzw. Bestandteile, insbesondere wie nachfolgend definiert - stets 100 % bzw. 100 Gew.-% resultieren. Dies versteht sich für den Fachmann aber von selbst.

Dies vorausgeschickt, wird im Folgenden die vorliegende Erfindung näher beschrieben.

Gegenstand der vorliegenden Erfindung - gemäß einem **ersten** Aspekt der vorliegenden Erfindung - ist somit eine monoterpenhaltige Darreichungsform für die perorale Applikation in Form einer Kapsel, insbesondere in Form einer Kapsel mit Kern/Hülle-Struktur mit mehrschichtiger Hülle, wobei die Kapsel von innen nach außen den folgenden Aufbau aufweist:
(a) einen Kapselkern, enthaltend mindestens eine monoterpenhaltige Wirksubstanz, insbesondere mindestens ein Monoterpen,
(b) eine erste, den Kapselkern umgebende, insbesondere den Kapselkern unmittelbar umgebende, strukturgebende und/oder formgebende Kapselhülle und
(c) eine zweite, die erste Kapselhülle umgebende, insbesondere die erste Kapselhülle unmittelbar umgebende, Kapselhülle, vorzugsweise in Form einer Beschichtung, insbesondere in Form einer magensaftresistenten, aber dünndarmlöslichen Beschichtung, wobei die zweite Kapselhülle mindestens zwei Komponenten (Inhaltsstoffe bzw. Bestandteile) umfasst, wobei eine erste Komponente ("Komponente 1") auf Basis von Alginsäure oder deren physiologisch verträglichen Salzen oder Estern und eine zweite Komponente ("Komponente 2") auf Cellulosebasis ausgebildet ist, wobei die Darreichungsform einen gewichtsbezogenen Anteil der zweiten Kapselhülle, bezogen die Darreichungsform, im Bereich von 0,5 bis 4 Gew.-% aufweist.

Mit anderen Worten besteht das Prinzip der vorliegenden Erfindung somit darin, eine Darreichungsform in Form einer Kapsel mit Kern/Hülle-Struktur bereitzustellen, welche einen inneren Kern, der eine monoterpenhaltige Wirksubstanz enthält, aufweist. Den Kapselkern umgibt eine erste Kapselhülle - entweder mittelbar oder vorzugsweise unmittelbar -, wobei diese erste Kapselhülle struktur- bzw. formgebend für die gesamte Darreichungsform ist, d. h. die erste Kapselhülle bestimmt zum einen die Form und somit die äußere Gestalt der Kapsel und zum anderen die grundlegenden mechanischen Eigenschaften der Darreichungsform. Die erste Kapselhülle wiederum wird von mindestens einer weiteren, der zweiten Kapselhülle umgeben - mittelbar oder vorzugsweise unmittelbar -, welche auch in Form einer Beschichtung ausgebildet sein kann und den Kapselkern sowie die erste Kapselhülle vor Umwelteinflüssen schützt; die zweite Kapselhülle kann dabei insbesondere die mechanischen Eigenschaften der ersten Kapselhülle und der Darreichungsform insgesamt modifizieren bzw. verbessern; insbesondere ermöglicht die zweite Kapselschicht eine gezielte Einstellung der mechanischen Eigenschaften der gesamten Darreichungsform. Die zweite Kapselhülle umfasst dabei - wie oben bereits ausgeführt - mindestens zwei Komponenten bzw. Inhaltsstoffe bzw. Bestandteile, wobei eine erste Komponente (die "Komponente 1 ") auf Basis von Alginsäure oder deren physiologisch verträglichen Salzen oder Estern ausgebildet ist und eine weitere, zweite Komponente ("Komponente 2") auf Cellulosebasis ausgebildet ist.

Im Allgemeinen ist es im Rahmen der vorliegenden Erfindung vorgesehen, dass die erste Kapselhülle sowohl magensaftlöslich als auch dünndarmlöslich ist und insbesondere allgemein in wässrigen Systemen löslich ist. Auf diese Weise ist es möglich, die Freisetzung der monoterpenhaltigen Wirksubstanz allein bzw. vorrangig durch die entsprechende Ausgestaltung bzw. Modifizierung der zweiten Kapselhülle zu steuern bzw. einzustellen.

Im Rahmen der vorliegenden Erfindung wird es bevorzugt, wenn die zweite Kapselhülle magensaftresistent, aber dünndarmlöslich ausgebildet ist, d. h. in Form einer sogenannten Retard-Beschichtung vorliegt. Eine derartige magensaftresistente, aber dünndarmlösliche Beschichtung bzw. Retard-Beschichtung besitzt den Vorteil, dass die im Kapselkern der Darreichungsform eingeschlossene monoterpenhaltige Wirksubstanz das aggressive, stark saure Milieu des Magens unbeschadet überstehen, im Dünndarm aber freigesetzt und resorbiert werden kann.

Unter dem Begriff "magensaftresistent" ist dabei im Rahmen der vorliegenden Erfindung insbesondere zu verstehen, dass die Kapseln mindestens zwei Stunden lang in 0,1 N Salzsäure, welche auf Temperaturen von 35 bis 39 °C erwärmt wird, unter ständiger Durchmischung aufbewahrt, insbesondere gerührt werden kann, ohne dass die Kapseln Anzeichen einer Zersetzung bzw. Risse oder andere Beschädigungen aufweisen.

Unter dem Begriff "dünndarmlöslich" ist im Rahmen der vorliegenden Erfindung hingegen insbesondere zu verstehen, dass die Kapseln in einer wässrigen Phosphatpuffer-Lösung, welche auf einen pH-Wert von etwa 6,8 eingestellt, unter Rühren bei Temperaturen im Bereich von 35 bis 39 °C innerhalb einer Stunde soweit zersetzt werden bzw. reißen, dass die Wirksubstanz freigesetzt wird.

Die im Rahmen dieser Erfindung verwendeten Definition für die Begriffe "magensaftresistent" bzw. "dünndarmlöslich" sowie die diesbezüglichen Testmethoden sind in European Pharmacopoeia 7.0, 04/2010: 1502, Seiten 707 bis 709, Stichwort "Capsules", Unterkapitel "Gastro-Resistant Capsules" sowie European Pharmacopoeia 7.1, 04/2011: 20901, Seiten 3331 und 3332, Stichwort "2.9.1 Disintegration of Tablets and Capsules*"* wiedergegeben.

Im Rahmen der vorliegenden Erfindung ist es somit gelungen, eine effiziente, gegenüber dem Stand der Technik deutlich verbesserte Darreichungsform für die perorale Applikation monoterpenhaltiger Wirksubstanzen bereitzustellen.

Durch die erfindungsgemäße Darreichungsform in Form einer Kapsel mit Kern/Hülle-Struktur wird eine Darreichungsform bereitgestellt, welche einerseits die monoterpenhaltige Wirksubstanz vor Umwelteinflüssen schützt und andererseits gewährleistet, dass die Wirksubstanz im Dünndarm unter kontrollierten Bedingungen freigesetzt wird.

Insbesondere die zweite Kapselhülle auf Basis von Alginsäure bzw. deren physiologisch verträglichen Salzen oder Estern einerseits sowie Cellulose bzw. Cellulosederivaten andererseits ermöglicht eine im Vergleich zum Stand der Technik deutlich einfachere, insbesondere apparativ weniger aufwendige, sowie zeitsparende und kostengünstigere Herstellung von monoterpenhaltigen Darreichungsformen für die systemische Anwendung. So ist beispielsweise die Verarbeitungsdauer (Prozesszeit) für die Ausbildung der Kapselhülle, insbesondere der zweiten Kapselhülle, d. h. die Prozessdauer für die eigentliche Befilmung, gegenüber Kapselhüllen des Standes der Technik, insbesondere auf (Meth-)Acrylsäure- bzw. (Meth-)Acrylatbasis, signifikant reduziert, insbesondere um mindestens etwa 25 %.

Die im Rahmen der vorliegenden Erfindung eingesetzte zweite Kapselhülle haftet deutlich besser an der ersten Kapselhülle, als dies für vergleichbare Kapseln des Standes der Technik beobachtet wird, so dass die Kapseln einerseits mechanisch widerstandsfähiger sind und andererseits auch kein Ablösen der zweiten Kapselhülle von der ersten Kapselhülle beobachtet wird, welches im Stand der Technik zu Eintrübungen der Kapselhülle bzw. zu opaken Flecken führt, welche wiederum vom Handel sowie von den Endverbrauchern beanstandet werden.

Durch die zweikomponentige Ausbildung der zweiten Kapselhülle mit einer ersten Komponente auf Basis von Alginsäure bzw. deren physiologisch verträglichen Salzen oder Estern einerseits und einer zweiten Komponente auf Cellulosebasis andererseits wird ein besonders optimales Freisetzungsprofil erreicht, da die zweite Kapselhülle sozusagen schaltbar ist: Im sauren Magensaftmilieu ist die zweite Kapselhülle unlöslich, insbesondere da die Alginsäurekomponente als freie Säure vorliegt und folglich unlöslich ist; im neutralen bis basischen Milieu dagegen, insbesondere im Milieu des Dünndarms, dagegen, wird der Alginsäureanteil der zweiten Kapselhülle zu Alginat umgewandelt, welches wiederum löslich, insbesondere wasserlöslich, ausgebildet ist, so dass dann die zweite Kapselhülle aufgelöst wird und letztendlich der Wirkstoff freigesetzt wird. Durch diese zweikomponentige Ausbildung der zweiten Kapselhülle wird also ein optimales Freisetzungsprofil erzielt.

Ein weiterer Vorteil der besonderen Ausgestaltung der zweiten Kapselhülle ist darin zu sehen, dass durch die Variation der Dicke der zweiten Kapselhülle, insbesondere durch Variation der Auftragmenge bei der Herstellung, der Zerfall der Kapsel und damit die Freisetzung des Wirkstoffs maßgeschneidert bzw. gezielt gesteuert werden kann.

Auch durch die Variation des Mengenverhältnisses der beiden Komponenten der zweiten Kapselhülle (d. h. Alginsäure bzw. Alginat einerseits und Cellulose bzw. Cellulosederivat andererseits) kann letztlich das Freisetzungsprofil der erfindungsgemäßen Darreichungsform gezielt gesteuert bzw. maßgeschneidert werden.

Weiterhin zeigen die Stabilitätsuntersuchungen der Anmelderin, dass im Rahmen der vorliegenden Erfindung, insbesondere infolge der speziell ausgebildeten zweiten Kapselhülle, keinerlei Interaktionen mit dem verkapselten Wirkstoff eintreten, d. h. der Kapselkern einerseits und die Kapselhüllen andererseits sind vollständig kompatibel zueinander ausgebildet.

Die erfindungsgemäße Darreichungsform weist im Vergleich zu Kapseln des Standes der Technik eine deutlich verbesserte Langzeitstabilität, insbesondere Lagerstabilität, auf, welche einerseits auf eine verbesserte Abschirmung des Kapselkerns sowie der monoterpenhaltigen Wirksubstanz gegenüber Umwelteinflüssen, wie der Einwirkung von Licht oder Sauerstoff, zurückzuführen ist und andererseits auch auf einer verbesserten Kompatibilität zwischen den Bestandteilen des Kapselkerns sowie der den Kapselkern umgebenden Kapselhüllen beruht. Des Weiteren weist die erfindungsgemäße Darreichungsform ein verbessertes Freisetzungsprofil auf.

Was die monoterpenhaltige Wirksubstanz anbelangt, welche im Rahmen der vorliegenden Erfindung eingesetzt wird, so kann diese aus einer Vielzahl von Monoterpenen ausgewählt werden. Im Rahmen der vorliegenden Erfindung wird es jedoch bevorzugt, wenn die monoterpenhaltige Wirksubstanz, insbesondere das Monoterpen, ausgewählt ist aus der Gruppe von 1,8-Cineol, Menthol, Campher, Limonen, Carvon, Carveol oder deren Kombinationen. Die vorgenannten Monoterpene können in hervorragender Weise den erfindungsgemäßen Darreichungsformen eingesetzt werden und besitzen darüber hinaus allesamt eine systemische pharmakologische Wirkung.

Besonders gute Ergebnisse werden im Rahmen der vorliegenden Erfindung erhalten, wenn das Monoterpen 1,8-Cineol und/oder Menthol, besonders bevorzugt 1,8-Cineol, ist.

Im Rahmen der vorliegenden Erfindung kann es vorgesehen sein, dass die Darreichungsform, insbesondere der Kapselkern, 1,8-Cineol als alleinigen Wirkstoff enthält oder aber die Darreichungsform, insbesondere der Kapselkern, 1,8-Cineol mit mindestens einem weiteren Wirkstoff, bevorzugt ausgewählt aus Terpenen, enthält.

Das im Rahmen der vorliegenden Erfindung eingesetzte Monoterpen, insbesondere 1,8-Cineol, kann somit als alleinige Wirksubstanz oder aber als Gemisch mit weiteren Substanzen, insbesondere als in der Natur vorkommendes Gemisch, wie beispielsweise Eukalyptusöl bzw. dessen Destillate, eingesetzt werden. Im Rahmen der vorliegenden Erfindung können somit Gemische von Naturstoffen bzw. deren gereinigte bzw. chemisch umgesetzte Produkte und Derivate unmittelbar eingesetzt werden.

Bevorzugt wird es jedoch, wenn die entsprechende monoterpenhaltige Wirksubstanz, insbesondere das Monoterpen, vorzugsweise 1,8-Cineol, in Form einer Reinsubstanz, d. h. als alleiniger isolierter Wirkstoff eingesetzt wird. Auf diese Weise kann beispielsweise eine immer gleichbleibende Dosierung bzw. ein immer gleicher Gehalt an Wirksubstanz in der erfindungsgemäßen Darreichungsform gewährleistet werden.

Was den terpenhaltigen bzw. terpenbasierten Wirkstoff, insbesondere speziell den Wirkstoff 1,8-Cineol, anbelangt, so kann bzw. können die erfindungsgemäße Darreichungsform bzw. die Innenkapseln 1,8-Cineol als alleinigen Wirkstoff enthalten oder aber - gemäß einer alternativen, aber weniger bevorzugten Ausführungsform - den Wirkstoff 1,8-Cineol zumindest mit mindestens einem weiteren Wirkstoff, bevorzugt ausgewählt aus Terpenen, enthalten. Erfindungsgemäß bevorzugt ist es, wenn die erfindungsgemäße Darreichungsform bzw. die Innenkapseln 1,8-Cineol als alleinigen Wirkstoff enthalten.

Dabei kann es im Rahmen der vorliegenden Erfindung vorgesehen sein, dass die monterpenhaltige Wirksubstanz, insbesondere das Monoterpen, allein den Kapselkern bildet oder aber vorzugsweise mit weiteren Hilfsstoffen bzw. Additiven den Kapselkern bildet.

Gemäß einer besonderen Ausführungsform enthält die Darreichungsform, insbesondere der Kapselkern, die monoterpenhaltige Wirksubstanz und/oder das Monoterpen, insbesondere 1,8-Cineol, zusammen mit mindestens einem mit der monoterpenhaltigen Wirksubstanz und/oder dem Monoterpen, insbesondere 1,8-Cineol, mischbaren und/oder hierin löslichen, insbesondere bei 20 °C und Atmosphärendruck flüssigen, physiologisch unbedenklichen Träger (Exzipienten). Der Träger bzw. Exzipient sollte selbst pharmakologisch nicht wirksam sein, aber mit der monoterpenhaltigen Wirksubstanz und/oder dem Monoterpen, insbesondere dem Wirkstoff 1,8-Cineol, eine vorzugsweise homogene Mischung oder Lösung bilden.

Gemäß einer erfindungsgemäß bevorzugten Ausführungsform kann der Träger bzw. Exzipient ausgewählt sein aus der Gruppe von fetten Ölen, bevorzugt Triglyceriden, besonders bevorzugt mittelkettigen Triglyceriden (*Medium Chain Triglycerides,* MCT), ganz besonders bevorzugt Triglyceriden mit C₆-C ₁₂-Fettsäureresten.

Erfindungsgemäß bevorzugt ist es, wenn der Träger bzw. Exzipient in einem monoterpenhaltigen Wirksubstanz/Träger-Mengenverhältnis im Bereich von 1.000 : 1 bis 1 : 1.000, insbesondere 100 : 1 bis 1 : 100, vorzugsweise 50 : 1 bis 1 : 50, besonders bevorzugt 10 : 1 bis 1 : 10, ganz besonders bevorzugt 5 : 1 bis 1 : 2, noch mehr bevorzugt 3 : 1 bis 1 : 1, eingesetzt wird. Durch die Mischung der monoterpenhaltigen Wirksubstanz mit den Exzipienten werden eine deutlich verbesserte Kompatibilität der monoterpenhaltigen Wirksubstanz mit den weiteren Materialien der Kapsel und eine verbesserte Stabilität, insbesondere Lagerstabilität, erreicht.

Der Begriff der Öle, wie er erfindungsgemäß für die vorstehenden Träger bzw. Exzipienten verwendet wird, ist eine Sammelbezeichnung für Flüssigkeiten, welche sich nicht mit Wasser mischen lassen. Der Begriff der fetten Öle, wie er in diesem Zusammenhang erfindungsgemäß verwendet wird, bezeichnet speziell Fette, d. h. Gemische von Fettsäuretriglyceriden, welche bei Raumtemperatur (insbesondere 20 °C) und Atmosphärendruck flüssig sind; insbesondere bezeichnet dieser Begriff Ester des dreiwertigen Alkohols Glycerin (Propan-1,2-3-triol) mit drei, meist verschiedenen, überwiegend geradzahligen und unverzweigten aliphatischen Monocarbonsäuren, den so genannten Fettsäuren. Verbindungen dieser Art werden auch Triglyceride genannt (nach IUPAC-Empfehlung: Triacylglycerine). Triglyceride, synonym auch als Glycerol-Triester bezeichnet, sind also dreifache Ester des dreiwertigen Alkohols Glycerin mit drei Säuremolekülen, wobei die Vorsilbe *"tri"* auf drei Acyl-Säurereste, die mit Glycerin verestert sind, verweist.

Speziell mittelkettige Triglyceride, wie sie erfindungsgemäß bevorzugt als Träger bzw. Exzipient für die monoterpenhaltige Wirksubstanz zum Einsatz kommen, sind insbesondere halbsynthetische neutrale Glycerinester von gesättigten, im Allgemeinen unverzweigten Monocarbonsäuren mittlerer Kettenlänge (d. h. C₆-C₁₂). Insbesondere bezeichnet der Begriff der mittelkettigen Triglyceride Gemische von Triglyceriden gesättigter Fettsäuren, hauptsächlich Caprylsäure (Octansäure) und Caprinsäure (Decansäure). Mittelkettige Triglyceride können im Allgemeinen aus Öl hergestellt werden, das aus dem festen und getrockneten Teil des Endosperms von *Cocos nucifera* L. und/oder aus dem getrockneten Endosperm von *Elaeis guineenses* Jacq. extrahiert wird. Für weitergehende Einzelheiten zu dem Begriff der mittelkettigen Triglyceride kann beispielsweise verwiesen werden auf die Monographie Ph. Eur., 6. Ausgabe, Grundwerk 2008, Seiten 4224 bis 4226, sowie auf die Zeitschrift für Ernährungswissenschaft, Band 13, Heft 1/2, 1973, Seiten 6 ff., D. Sailer et al. "Mittelkettige Triglyceride - Klinische Physiologie und Anwendung*").*

Der Gehalt an monoterpenhaltiger Wirksubstanz in der erfindungsgemäßen Darreichungsform kann gleichermaßen in weiten Grenzen variieren: Üblicherweise enthält die erfindungsgemäße Darreichungsform den Wirkstoff, insbesondere 1,8-Cineol, in absoluten Mengen von 10 bis 1.000 mg, insbesondere 25 bis 750 mg, vorzugsweise 50 bis 500 mg, besonders bevorzugt 75 bis 300 mg, bezogen auf eine Applikationseinheit, insbesondere Kapsel.

Unter einer Applikationseinheit soll dabei im Rahmen der vorliegenden Erfindung insbesondere eine genormte Dosis der Wirksubstanz verstanden werden, welche zu unmittelbaren Anwendungen hergerichtet ist. Eine Applikationseinheit im Sinne der vorliegenden Erfindung ist beispielsweise ein Dragee, eine Tablette oder eine Kapsel.

Im Allgemeinen enthält die erfindungsgemäße Darreichungsform den Wirkstoff, insbesondere 1,8-Cineol, in relativen Mengen von 0,01 bis 99 Gew.-%, insbesondere 0,1 bis 95 Gew.-%, vorzugsweise 1 bis 90 Gew.-%, besonders bevorzugt 5 bis 80 Gew.-%, bezogen auf die Darreichungsform.

Besonders gute Ergebnisse werden im Rahmen der vorliegenden Erfindung erhalten, wenn die Darreichungsform den Kapselkern in Mengen von 10 bis 1.500 mg, insbesondere 50 bis 1.250 mg, vorzugsweise 75 bis 1.000 mg, bevorzugt 100 bis 750 mg, besonders bevorzugt 125 bis 500 mg, pro Kapsel bzw. Darreichungsform enthält.

Gleichfalls kann es vorgesehen sein, dass die Darreichungsform einen gewichtsbezogenen Anteil des Kapselkerns, bezogen die Darreichungsform, im Bereich von 10 bis 90 Gew.-%, insbesondere 20 bis 85 Gew.-%, vorzugsweise 40 bis 80 Gew.-%, bevorzugt 50 bis 75 Gew.-%, besonders bevorzugt 60 bis 70 Gew.-%, aufweist.

Was weiterhin die erste Kapselhülle der erfindungsgemäßen Darreichungsform anbelangt, so kann diese aus einer Vielzahl von Materialien bestehen.

Besonders gute Ergebnisse werden im Rahmen der vorliegenden Erfindung jedoch erhalten, wenn die erste Kapselhülle als Weichkapsel bzw. Weichkapselhülle ausgebildet ist.

Zu diesem Zweck kann es vorgesehen sein, dass das Kapselhüllenmaterial der ersten Kapselhülle mindestens ein pharmakologisch unbedenkliches Polymer umfasst oder hieraus besteht. Vorzugsweise wird dabei das pharmakologisch unbedenkliche Polymer ausgewählt aus synthetischen, natürlichen oder naturidentischen Polymeren und Polymerisaten.

Bevorzugt wird es im Rahmen der vorliegenden Erfindung darüber hinaus, wenn das mindestens eine pharmakologisch unbedenkliche Polymer ausgewählt ist aus der Gruppe von Proteinen, vorzugsweise Gelatine und/oder Caseinaten, bevorzugt Gelatine, sowie deren Mischungen und Kombinationen. Besonders gute Ergebnisse werden im Rahmen der vorliegenden Erfindung erhalten, wenn das Kapselhüllenmaterial der ersten Kapselhülle Gelatine, insbesondere Hart- oder Weichgelatine, vorzugsweise Weichgelatine, umfasst oder hieraus besteht.

In diesem Zusammenhang kann es vorgesehen sein, dass die Gelatine zusammen mit mindestens einem weiteren Additiv oder Hilfsstoff eingesetzt wird. Wenn die Gelatine zusammen mit mindestens einem weiteren Additiv oder Hilfsstoff eingesetzt wird, so hat es sich bewährt, wenn das weitere Additiv oder der weitere Hilfsstoff ausgewählt ist aus Weichmachern, Feuchthaltemitteln und Schmiermitteln sowie deren Mischungen und Kombinationen. Vorzugsweise beträgt in diesem Fall die Menge an Additiv oder Hilfsstoff insgesamt 0,01 bis 40 Gew.-%, insbesondere 0,1 bis 35 Gew.-%, vorzugsweise 1 bis 30 Gew.-%, bezogen auf die erste Kapselhülle.

Die Auswahl von Weichgelatine als erstem Kapselhüllenmaterial hat den Vorteil, dass bei geeigneter Wahl des Exzipienten bzw. Trägermaterials für den Kapselkern keine Wechselwirkung zwischen der Wirksubstanz und der Gelatine zu erwarten sind bzw. von der Anmelderin nicht beobachtet wurden. Darüber hinaus ist Gelatine als Lebensmittel zugelassen und physiologisch verträglich, gesundheitlich unbedenklich und besitzt hervorragende mechanische Eigenschaften, insbesondere wenn flüssige nicht-wässrige Substanzen verkapselt werden.

Darüber hinaus erzeugen Weichgelatinekapseln einen angenehmeren haptischen Eindruck im Vergleich zu Hartkapseln und werden aufgrund der hohen Oberflächenglätte sowie der Verformbarkeit vom Anwender eher akzeptiert als Hartkapseln.

Im Allgemeinen enthält die erfindungsgemäße Darreichungsform die erste Kapselhülle in Mengen von 40 bis 500 mg, insbesondere 50 bis 400 mg, vorzugsweise 60 bis 300 mg, bevorzugt 70 bis 250 mg, besonders bevorzugt 80 bis 200 mg, pro Darreichungsform bzw. Kapsel.

Weiterhin kann es vorgesehen sein, dass die Darreichungsform einen gewichtsbezogenen Anteil der ersten Kapselhülle, bezogen auf die Darreichungsform, im Bereich von 15 bis 75 Gew.-%, insbesondere 20 bis 65 Gew.-%, vorzugsweise 25 bis 55 Gew.-%, bevorzugt 30 bis 50 Gew.-%, besonders bevorzugt 35 bis 45 Gew.-%, aufweist.

Bei den vorgenannten absoluten Mengen bzw. relativen Mengenanteilen für die erste Kapselhülle wird gewährleistet, dass die erste Kapselhülle insgesamt hervorragende mechanische Eigenschaften besitzt und den Kapselkern vor unerwünschten mechanischen und chemischen Einflüssen schützt, wobei gleichzeitig eine kontrollierte reproduzierbare Freisetzung der Wirksubstanz erreicht werden kann.

Was die Materialien der zweiten Kapselhülle anbelangt, so können diese gleichfalls im Rahmen der vorliegenden Erfindung in weiten Bereichen variieren. Besonders gute Ergebnisse werden jedoch erzielt, wenn die zweite Kapselhülle als erste Komponente ("Komponente 1") mindestens ein Alginat aufweist, insbesondere ausgewählt aus Alkalimetallalginaten, Erdalkalimetallalginaten, Ammonium-alginaten und Alginsäureestern, vorzugsweise ausgewählt aus Natriumalginat, Kaliumalginat, Ammoniumalginat, Calciumalginat und Propylenglykolalginat, bevorzugt ausgewählt aus Natriumalginat, Kaliumalginat, Ammoniumalginat und Calciumalginat, besonders bevorzugt Natriumalginat.

Wie oben bereits ausgeführt, weist die zweite Kapselhülle neben der ersten Komponente auf Basis von Alginsäure bzw. deren physiologisch verträglichen Salzen oder Estern auch noch eine zweite Komponente auf Basis von Cellulose ("Komponente 2") auf. Im Rahmen der vorliegenden Erfindung wird dabei bevorzugt, wenn die zweite Kapselhülle als zweite Komponente Cellulose oder ein Cellulosederivat, insbesondere einen Celluloseether oder Celluloseester, enthält. In diesem Zusammenhang hat es sich besonders bewährt, wenn die zweite Komponente ausgewählt ist aus Celluloseethern, vorzugsweise aus der Gruppe von Methylcellulose, Ethylcellulose, Ethylmethylcellulose, Hydroxypropylcellulose, Hydroxypropylmethylcellulose, Carboxymethylcellulose oder deren Kombinationen, vorzugsweise Ethylcellulose.

Wie es sich im Rahmen der vorliegenden Erfindung gezeigt hat, werden die Eigenschaften der zweiten Kapselhülle nicht nur durch die Auswahl der Materialien (Alginsäure bzw. Alginat einerseits und Cellulose bzw. Cellulosederivate andererseits) bestimmt bzw. beeinflusst, sondern vielmehr auch durch deren mengenmäßiges Verhältnis.

Gemäß einer bevorzugten Ausführungsform der vorliegenden Erfindung weist die zweite Kapselhülle die erste und die zweite Komponente in einem gewichtsbezogenen Verhältnis von erster Komponente zu zweiter Komponente (Komponente 1/Komponente 2-Gewichtsverhältnis), vorzugsweise Gewichtsverhältnis Alginsäure bzw. Alginat/Cellulose bzw. Cellulosederivat, von 1 : 99 bis 50 : 50, insbesondere 5 : 95 bis 40 : 60, vorzugsweise 8 : 92 bis 30 : 70, bevorzugt 10 : 90 bis 25 : 75, besonders bevorzugt 12 : 88 bis 20 : 80, auf.

Die im Rahmen der vorliegenden Erfindung verwendete zweite Kapselhülle ist deutlich einfacher aufzubringen, d. h. mit deutlich weniger apparativem Aufwand sowie weniger Ausgangsstoffen als bislang im Stand der Technik übliche Kapselhüllen bzw. Beschichtungen; darüber hinaus ist die zweite Kapselhülle deutlich kostengünstiger und zeitökonomischer und mit höheren Durchsätzen auf die Kapseln aufzubringen, als dies für vergleichbare Kapseln im Stand der Technik möglich ist.

Insbesondere die Kombination von Alginsäure bzw. Alginsäuresalzen oder -estern einerseits und Cellulose bzw. Cellulosederivaten andererseits führt zu einer zweiten Kapselhülle bzw. Beschichtung, welche sich in hervorragender Weise als Retard-Beschichtung für eine kontrollierte und verzögerte Freisetzung der monoterpenhaltigen Wirksubstanz eignet. Die zweite Kapselhülle schützt die erste Kapselhülle sowie den Kapselkern zuverlässig vor Umwelteinflüssen, insbesondere dem Einfluss von Luftsauerstoff sowie vor wässrigen Säuren, wodurch eine Magensaftresistenz der erfindungsgemäßen Darreichungsform bzw. Kapsel erreicht werden kann. Andererseits kann die erfindungsgemäße Darreichungsform - insbesondere infolge der Anwesenheit der Alginsäure bzw. Alginate - in schwach basischen oder neutralen wässrigen Medien (d. h. im Dünndarm-Milieu) kontrolliert aufgelöst bzw. zersetzt werden, wodurch eine kontrollierte verzögerte Freisetzung der monoterpenhaltigen Wirksubstanz erreicht werden kann. Insbesondere in den zuvor genannten gewichtsbezogenen Verhältnissen von Alginsäure(derivat) zu Cellulose(derivat) kann eine besonders effektive Retard-Beschichtung erhalten werden.

Darüber hinaus haftet die erfindungsgemäß eingesetzte zweite Kapselhülle deutlich besser an der ersten Kapselhülle als vergleichbare Kapselhüllen bzw. Beschichtungen des Standes der Technik, welche im Allgemeinen auf Basis von (Meth-)Acrylsäurederivaten bzw. (Meth-)Acrylaten (z. B. Eudragit^{®}) ausgebildet sind.

Im Rahmen der vorliegenden Erfindung kann es auch vorgesehen sein, dass die zweite Kapselhülle neben Alginsäure(derivaten) und Cellulose(derivaten) noch weitere Bestandteile enthält. Insbesondere kann es vorgesehen sein, dass die zweite Kapselhülle mindestens einen weiteren Bestandteil oder Inhaltsstoff, insbesondere ausgewählt aus Additiven oder Hilfsstoffen, vorzugsweise aus fetten Ölen und/oder Fettsäuren, besonders bevorzugt ausgewählt aus der Gruppe von Stearinsäure, Ölsäure und Triglyceriden sowie deren Mischungen und Kombinationen, aufweist. In diesem Zusammenhang wird bevorzugt, wenn die Triglyceride aus mittelkettigen Triglyceriden (*Medium Chain Triglycerides,* MCT), vorzugsweise aus Triglyceriden mit C₆-C₁₂-Fettsäureresten, ausgewählt sind. Gleichfalls werden besonders gute Ergebnisse erhalten, wenn die Menge an weiterem Bestandteil oder Inhaltsstoff insgesamt 0,01 bis 35 Gew.-%, insbesondere 0,1 bis 30 Gew.-%, vorzugsweise 1 bis 25 Gew.-%, bezogen auf die zweite Kapselhülle, beträgt. Die zuvor genannten Hilfsstoffe und Additive werden vorrangig und insbesondere als Weichmacher verwendet, wodurch die zweite Kapselhülle der erfindungsgemäßen Darreichungsform und somit die Darreichungsform insgesamt eine höhere Flexibilität erhält und mechanischen Belastungen besser widerstehen kann. Darüber hinaus erhöhen die Additive und Hilfsstoffe nochmals die Kompatibilität der zweiten Kapselhülle mit der ersten Kapselhülle, insbesondere wenn diese auf Basis von Gelatine ausgebildet ist. Auf diese Weise wird die Langzeitstabilität der erfindungsgemäßen Darreichungsform gesteigert. Zur Steigerung der Kompatibilität zwischen erster und zweiter Kapselhülle entsprechen die der zweiten Kapselhülle zugesetzten Hilfsstoffe oder Additive weitestgehend den Hilfsstoffen bzw. Additiven des Kapselkerns.

Im Rahmen der Herstellung der erfindungsgemäße Darreichungsform hat es sich darüber hinaus als vorteilhaft erwiesen, wenn die zweite Kapselhülle basisch eingestellt ist, d. h. die zweite Kapselhülle einen basischen pH-Wert besitzt (pH > 7). Ein basischer pH-Wert ermöglicht die Aufbringung der erfindungsgemäß eingesetzten zweiten Kapselhülle in Form einer Dispersion oder Lösung auf die erste Kapselhülle.

Was die Mengen bzw. die gewichtsbezogenen Anteile der zweiten Kapselhülle in Bezug auf die Darreichungsform anbelangt, so hat es sich als vorteilhaft erwiesen, wenn die Darreichungsform die zweite Kapselhülle in Mengen von 0,5 bis 100 mg, insbesondere 1 bis 75 mg, vorzugsweise 1 bis 50 mg, bevorzugt 2 bis 40 mg, besonders bevorzugt 3 bis 35 mg, ganz besonders bevorzugt 5 bis 30 mg, enthält, bezogen auf die Darreichungsform.

Erfindungsgemäß werden besonders gute Ergebnisse erhalten, da die Darreichungsform einen gewichtsbezogenen Anteil der zweiten Kapselhülle, bezogen auf die Darreichungsform, im Bereich von 2 bis 4 Gew.-% aufweist.

Was die Abmessungen der Darreichungsform bzw. der Kapsel bzw. die Dicken der einzelnen Schichten bzw. Kapselhüllen anbelangt, so können diese im Rahmen der vorliegenden Erfindung in beiden Bereichen variieren. Besonders gute Ergebnisse werden jedoch erhalten, wenn der Durchmesser, insbesondere der mittlere Durchmesser, der Darreichungsform im Bereich von 50 µm bis 25 mm, insbesondere im Bereich von 100 µm bis 25 mm, vorzugsweise im Bereich von 250 µm bis 25 mm, besonders bevorzugt im Bereich von 500 µm bis 25 mm, ganz besonders bevorzugt im Bereich von 750 µm bis 25 mm, noch mehr bevorzugt im Bereich von 1.000 µm bis 25 mm, variiert. In diesen Bereichen können im Rahmen der vorliegenden Erfindung mechanisch stabile Kapseln, welche die monoterpenhaltige Wirksubstanz effektiv gegen Umwelteinflüsse schützen, hergestellt werden, wobei eine kontrollierte Freisetzung der Wirksubstanz, insbesondere eine retardierte Freisetzung der Wirksubstanz unter kontrollierten Bedingungen, erzielt werden kann. Darüber hinaus kann es im Rahmen der vorliegenden Erfindung vorgesehen sein, dass die erste Kapselhülle eine Dicke, insbesondere mittlere Dicke, im Bereich von 0,01 bis 8 mm, insbesondere 0,05 bis 7 mm, vorzugsweise 0,1 bis 6 mm, bevorzugt 0,2 bis 5 mm, besonders bevorzugt 0,5 bis 4 mm, ganz besonders bevorzugt 1 bis 3 mm, aufweist. Im Fall von ersten Kapselhüllen mit mittleren Dicken im vorgenannten Bereich können gute bis hervorragende mechanische Eigenschaften der Kapseln sowie eine kontrollierte Freisetzung der Wirksubstanz erzielt werden.

Weiterhin kann es im Rahmen der vorliegenden Erfindung vorgesehen sein, dass die zweite Kapselhülle eine Dicke, insbesondere mittlere Dicke, im Bereich von 0,01 bis 3 mm, insbesondere 0,05 bis 2,5 mm, vorzugsweise 0,07 bis 2 mm, bevorzugt 0,08 bis 1,5 mm, besonders bevorzugt 0,1 bis 1 mm, ganz besonders bevorzugt 0,2 bis 0,5 mm, aufweist. Eine zweite Kapselhülle mit einer Dicke im vorgenannten Bereich kann sowohl gleichfalls die mechanischen Eigenschaften der Darreichungsform insgesamt verstärken als auch die kontrollierte bzw. verzögerte Freisetzung der Wirksubstanz gewährleisten.

Im Rahmen der vorliegenden Erfindung werden besonders gute Ergebnisse erhalten, wenn das Verhältnis der Dicke der ersten Kapselhülle zur Dicke der zweiten Kapselhülle 500 : 1 bis 1 : 1, insbesondere 100 : 1 bis 2 : 1, vorzugsweise 50 : 1 bis 5 : 1, beträgt.

Darüber hinaus kann es vorgesehen sein, dass das gewichtsbezogene Verhältnis von erster Kapselhülle zu zweiter Kapselhülle 1 : 1 bis 50 : 1, insbesondere 2 : 1 bis 30 : 1, vorzugsweise 3 : 1 bis 25 : 1, bevorzugt 5 : 1 bis 20 : 1, beträgt.

Im Rahmen der vorliegenden Erfindung ist es somit möglich, die Dicke und Eigenschaften der ersten und der zweiten Kapselhülle gezielt auf den jeweiligen Anwendungsfall bzw. die jeweiligen Anwendungserfordernisse maßzuschneidern und die erfindungsgemäße Darreichungsform neuen Wirksubstanzen bzw. neuen Wirksubstanzkombinationen anzupassen.

Darüber hinaus ist die Oberfläche der zweiten Kapselhülle glatter als die Oberfläche vergleichbarer Kapselhüllen bzw. Beschichtungen des Standes der Technik, wodurch die erfindungsgemäße Darreichungsform sich weicher und glatter anfühlt und somit eine ansprechendere Haptik aufweist. Des Weiteren wird durch die glattere Oberfläche der zweiten Kapselhülle die erfindungsgemäße Darreichungsform besser anwendbar, da sie einfacher peroral zu applizieren bzw. einzunehmen ist und ein angenehmeres Gefühl für den Anwender beim Schlucken erzeugt.

Im Rahmen der vorliegenden Erfindung kann es weiterhin vorgesehen sein, dass die erfindungsgemäße Darreichungsform mit einer äußeren, die zweite Kapselhülle umgebenden, insbesondere die zweite Kapselhülle unmittelbar umgebenden, Beschichtung, insbesondere auf Basis von Lacken oder Wachsen sowie deren Mischungen und Kombinationen, versehen ist. Eine derartige weitere Beschichtung steigert die Langzeitstabilität der erfindungsgemäßen Darreichungsform nochmals beträchtlich und verbessert darüber hinaus die Retardierungseigenschaften; insbesondere werden jedoch die optischen Eigenschaften, wie beispielsweise der Glanz, sowie die Haptik verbessert, wodurch die erfindungsgemäße Darreichungsform als Ganzes ein angenehmeres Aussehen bekommt. Durch eine solche weitere Beschichtung, insbesondere mit Wachsen, wird darüber hinaus die Oberflächenglätte der erfindungsgemäßen Darreichungsform nochmals verbessert, wodurch die erfindungsgemäße Darreichungsform einfacher in größeren Verpackungseinheiten, wie beispielsweise Blisterverpackungen, eingefüllt werden kann und Fehlverpackungen vermieden werden, d. h. diese Beschichtung insbesondere auf Basis von Lacken oder Wachsen hat nicht nur optische Vorteile, sondern verbessert in signifikanter Weise auch das Gleitverhalten der erfindungsgemäßen Darreichungsform (z. B. beim Befüllen der Blisterhöfe von Blisterpackungen), so dass etwaige Störungen bei der Verpackung bzw. Konfektionierung vermieden werden. Besonders gute Ergebnisse werden im Rahmen der vorliegenden Erfindung erhalten, wenn die Darreichungsform die optional vorhandene äußere Beschichtung in Mengen von 0,001 bis 20 mg, insbesondere 0,01 bis 10 mg, vorzugsweise 0,1 bis 1 mg, bevorzugt 0,2 bis 0,5 mg, aufweist. Gleichfalls kann es vorgesehen sein, dass die Darreichungsform einen gewichtsbezogenen Anteil der optionalen äußeren Beschichtung, bezogen auf die Darreichungsform, von 0,01 bis 1 Gew.-%, insbesondere 0,01 bis 0,5 Gew.-%, vorzugsweise 0,02 bis 0,3 Gew.-%, bevorzugt 0,05 bis 0,15 Gew.-%, aufweist. Gemäß einer bevorzugten Ausführungsform der vorliegenden Erfindung kann die äußere Beschichtung insbesondere auf Basis von Wachsen ausgebildet sein, insbesondere auf Basis von Candelillawachs, Carnaubawachs oder Bienenwachs. Besonders bevorzugt wird es dabei im Rahmen der vorliegenden Erfindung, wenn die äußere Beschichtung aus Candelillawachs besteht.

Wie oben bereits erwähnt, zeichnet sich die erfindungsgemäße Darreichungsform durch eine ausgezeichnete Langzeit- bzw. Lagerstabilität aus.

Im Rahmen der vorliegenden Erfindung kann es dabei vorgesehen sein, dass die Darreichungsform bei einer Temperatur im Bereich von 25 °C bis 40 °C und bei einer relativen Luftfeuchte von 50 % bis 80 % mindestens 6 Monate, insbesondere mindestens 12 Monate, vorzugsweise mindestens 18 Monate, bevorzugt mindestens 24 Monate, besonders bevorzugt mindestens 36 Monate, lagerstabil ist, insbesondere gemäß ICH-Standard (**I**nternational **C**onference on **H**armonisation of Technical Requirements for Registration of Pharmaceuticals for Human Use) lagerstabil ist.

In diesem Zusammenhang kann es insbesondere vorgesehen sein, dass die Darreichungsform bei einer Temperatur von 40 °C und bei einer relativen Luftfeuchte von 75 % mindestens 6 Monate lagerstabil ist. Insbesondere kann es vorgesehen sein, dass die Darreichungsform bei einer Temperatur von 30 °C und bei einer relativen Luftfeuchte von 65 % mindestens 12 Monate lagerstabil ist. Vorzugsweise kann es vorgesehen sein, dass die Darreichungsform bei einer Temperatur von 25 °C und bei einer relativen Luftfeuchte von 60 % mindestens 36 Monate lagerstabil ist.

Darüber hinaus wird es im Rahmen der vorliegenden Erfindung bevorzugt, wenn die Darreichungsform bei Einwirkung von 0,1 N Salzsäure bei 37 °C und unter ständiger Durchmischung, insbesondere unter Rühren, über einen Zeitraum von 2 Stunden zumindest im Wesentlichen unzersetzt bleibt, insbesondere strukturell intakt bleibt.

Gleichfalls ist es bevorzugt, wenn die Darreichungsform bei Einwirkung eines Phosphatpuffers mit einem pH-Wert von etwa 6,8 bei 37 °C und unter ständiger Umwälzung bzw. Durchmischung, insbesondere unter Rühren, über einen Zeitraum von einer Stunde zumindest im Wesentlichen zersetzt bleibt, insbesondere vollständig zersetzt ist.

Die erfindungsgemäße Darreichungsform eignet sich für eine Vielzahl von Verwendungen, insbesondere zur Herstellung von Arzneimitteln, wie im Folgenden noch dargelegt wird.

Weitere vorteilhafte Eigenschaften, Aspekte und Merkmale der vorliegenden Erfindung ergeben sich aus der folgenden Beschreibung eines in den Figuren dargestellten Ausführungsbeispiels. Es zeigt:

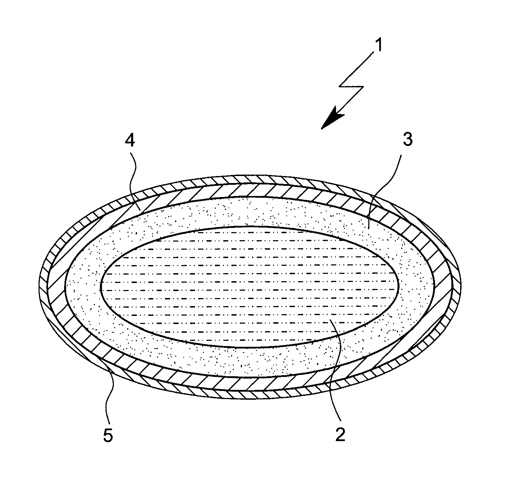

- Fig.1: eine schematische Schnittdarstellung durch eine erfindungsgemäße monoterpenhaltige Darreichungsform gemäß einer Ausführungsform der vorliegenden Erfindung.

Die einzige Figur zeigt eine erfindungsgemäße, für die perorale Applikation bestimmte, in Form einer Kapsel ausgebildete monoterpenhaltige Darreichungsform 1 gemäß einer Ausführungsform der vorliegenden Erfindung. Wie sich aus der Figurendarstellung ergibt, ist die Darreichungsform 1 als Kapsel mit Kern/Hülle-Struktur ausgebildet, wobei die Darreichungsform 1 einen monoterpenhaltigen Kapselkern 2 und eine diesen Kapselkern 2 umgebende erste Kapselhülle 3 aufweist. Die erste Kapselhülle 3 wiederum ist von einer zweiten Kapselhülle bzw. Beschichtung 4 umgeben, wobei auf die zweite Kapselhülle bzw. Beschichtung 4 eine weitere äußere Beschichtung 5 (z. B. Wachs) aufgebracht sein kann.

Für weitere Einzelheiten zu der in der Figurendarstellung dargestellten Ausführungsform kann zur Vermeidung unnötiger Wiederholungen auf die obigen Ausführungen verwiesen werden, welche in Bezug auf die Figurendarstellung entsprechend gelten.

Weiterer Gegenstand der vorliegenden Erfindung - gemäß einem **zweiten** Aspekt der vorliegenden Erfindung - ist ein Verfahren zur Herstellung einer monoterpenhaltigem Darreichungsform für die perorale Applikation in Form einer Kapsel, insbesondere in Form einer Kapsel mit Kern/Hülle-Struktur mit mehrschichtiger Hülle, wie sie zuvor beschrieben wurde, wobei
(i) in einem ersten Verfahrensschritt ein Kapselkern, enthaltend mindestens eine monoterpenhaltige Wirksubstanz, insbesondere mindestens ein Monoterpen, mit einer ersten, den Kapselkern umgebenden, insbesondere den Kapselkern unmittelbar umgebenden, strukturgebenden und/oder formgebenden Kapselhülle versehen wird und
(ii) in einem nachfolgenden zweiten Verfahrensschritt die erste Kapselhülle mit einer zweiten, die erste Kapselhülle umgebenden, insbesondere die erste Kapselhülle unmittelbar umgebenden, Kapselhülle, vorzugsweise in Form einer Beschichtung, insbesondere in Form einer magensaftresistenten, aber dünndarmlöslichen Beschichtung, versehen wird, wobei zur Herstellung der zweiten Kapselhülle mindestens zwei Komponenten eingesetzt werden, wobei eine erste Komponente ("Komponente 1 ") auf Basis von Alginsäure oder deren physiologisch verträglichen Salzen oder Estern und eine zweite Komponente ("Komponente 2") auf Cellulosebasis ausgebildet ist, wobei die erste und die zweite Komponente zur Herstellung der zweiten Kapselhülle gemeinsam in Form einer wässrigen Lösung und/oder Dispersion aufgebracht werden, wobei die Lösung und/oder Dispersion basisch eingestellt wird und wobei die erhaltene Darreichungsform einen gewichtsbezogenen Anteil der zweiten Kapselhülle, bezogen die Darreichungsform, im Bereich von 0,5 bis 4 Gew.-% aufweist.

Verfahrensschritt (i) wird dabei mit im Stand der Technik bekannten Verfahren durchgeführt. So kann beispielsweise bei Verwendung von Weichgelatine als erster Kapselhülle die erste Kapselhülle mit dem darin eingeschlossenen Kapselkern nach dem *Rotary-Die-Verfahren* hergestellt werden, welches dem Fachmann als solches geläufig ist und keiner weiteren Erläuterung bedarf.

Im Rahmen des erfindungsgemäßen Verfahrens ist es darüber hinaus vorgesehen, dass die erste und die zweite Komponente zur Herstellung der zweiten Kapselhülle gemeinsam in Form einer wässrigen Lösung und/oder Dispersion aufgebracht werden.

Auf diese Weise wird eine besonders homogene und gute Durchmischung der beiden Komponenten erreicht und somit eine gleichmäßige Beschichtung erhalten, welche eine kontrollierte Freisetzung der monoterpenhaltigen Wirksubstanz erlaubt.

Darüber hinaus ist es im Rahmen der vorliegenden Erfindung vorgesehen, dass die Lösung und/oder Dispersion basisch eingestellt wird, insbesondere mit wässriger Ammoniaklösung. Diese Vorgehensweise ist insbesondere bei Verwendung von Alginat als erster Komponente der zweiten Kapselhülle bevorzugt, da auf diese Weise eine gute Löslichkeit bzw. Dispergierbarkeit des Alginats im wässrigen Medium erreicht wird.

Was die Aufbringung der Lösung bzw. Dispersion zur Herstellung der zweiten Kapselhülle betrifft, so kann diese durch verschiedene Verfahren und unter unterschiedlichsten Verfahrensbedingungen durchgeführt werden.

Besonders gute Ergebnisse werden jedoch im Rahmen der vorliegenden Erfindung erhalten, wenn die Lösung und/oder Dispersion bei Temperaturen im Bereich von 10 bis 70 °C, insbesondere 15 bis 60 °C, vorzugsweise 20 bis 50 °C, bevorzugt 25 bis 40 °C, aufgebracht wird. Es ist eine Besonderheit des erfindungsgemäßen Verfahrens, dass der Auftrag der Lösung und/oder Dispersion, welche die beiden Komponenten zur Herstellung der zweiten Kapselhülle enthält, auch bei Raumtemperatur erfolgen kann und dabei eine stabile, insbesondere langzeitstabile und mechanisch belastbare zweite Kapselhülle bzw. Beschichtung erhalten wird.

Bei der Aufbringung der Lösung und/oder Dispersion zur Herstellung der zweiten Kapselhülle wird es darüber hinaus bevorzugt, wenn die Lösung bzw. Dispersion einen Feststoffanteil von 10 bis 25 Gew.-% aufweist.

Im Allgemeinen wird die Lösung und/oder Dispersion mittels Tauch- und/oder Sprühverfahren, insbesondere mittels Sprühverfahren, aufgebracht. Ein solcher Sprühauftrag kann beispielsweise mittels eines sogenannten *Pan- Coater* erfolgen, in welchem üblicherweise mehrere tausend Kapseln und bei großindustrieller Herstellung bis zu mehreren Hunderttausend und sogar bis zu mehreren Millionen Kapseln unter ständiger Umwälzung kontinuierlich mit einer Beschichtungslösung besprüht werden.

Darüber hinaus kann es im Rahmen der vorliegenden Erfindung vorgesehen sein, dass im Rahmen des erfindungsgemäßen Verfahrens noch ein abschließender Verfahrensschritt (iii) vorgesehen ist. Insbesondere kann es in diesem Zusammenhang vorgesehen sein, dass in einem abschließenden Schritt (iii) mindestens eine Beschichtung, insbesondere auf Basis von Lacken oder Wachsen sowie deren Mischungen und Kombinationen, auf die zweite Kapselhülle aufgebracht wird, insbesondere unmittelbar auf die zweite Kapselhülle aufgebracht wird. Die Aufbringung einer weiteren Beschichtung in einem Verfahrensschritt (iii) verbessert insbesondere die Oberflächeneigenschaften der erfindungsgemäßen Darreichungsformen nochmals beträchtlich.

Für weitergehende Einzelheiten zu dem erfindungsgemäßen Verfahren kann auf die obigen Ausführungen zu der erfindungsgemäßen Darreichungsform verwiesen werden, welche in Bezug auf das erfindungsgemäße Verfahren entsprechend gelten.

Wie zuvor bereits beschrieben, ist somit die monoterpenhaltige Darreichungsform nach der vorliegenden Erfindung zur Verwendung in der Humanmedizin oder Veterinärmedizin bzw. zur prophylaktischen und/oder therapeutischen Behandlung des menschlichen oder tierischen Körpers geeignet.

So kann die erfindungsgemäße monoterpenhaltige Darreichungsform beispielsweise zur prophylaktischen und/oder therapeutischen Behandlung von entzündlichen, infektexazerbierten oder allergischen Erkrankungen des menschlichen oder tierischen Körpers bzw. zur Herstellung eines Arzneimittels zur prophylaktischen und/oder therapeutischen Behandlung von entzündlichen, infektexazerbierten oder allergischen Erkrankungen des menschlichen oder tierischen Körpers verwendet werden.

Gleichermaßen kann beispielsweise die erfindungsgemäße monoterpenhaltige Darreichungsform zur prophylaktischen und/oder therapeutischen Behandlung bzw. zur Herstellung eines Arzneimittels zur prophylaktischen und/oder therapeutischen Behandlung von Autoimmunerkrankungen des menschlichen oder tierischen Körpers angewendet werden.

Weiterhin kann die erfindungsgemäße monoterpenhaltige Darreichungsform zur prophylaktischen und/oder therapeutischen Behandlung bzw. zur Herstellung eines Arzneimittels zur prophylaktischen und/oder therapeutischen Behandlung von Erkältungskrankheiten und grippalen Infekten und hiermit im Zusammenhang stehenden Infekten und Erkrankungen, insbesondere Infekten der oberen und unteren Atemwege, insbesondere Rhinitis, Sinusitis und bronchopulmonalen Erkrankungen, verwendet werden.

Weiterhin kann die erfindungsgemäße monoterpenhaltige Darreichungsform zur prophylaktischen und/oder therapeutischen Behandlung bzw. zur Herstellung eines Arzneimittels zur prophylaktischen und/oder therapeutischen Behandlung von Atemwegserkrankungen, insbesondere von bronchopulmonalen Erkrankungen, insbesondere Bronchitis, Asthma bronchiale und chronischen obstruktiven Lungenerkrankungen (COPD), verwendet werden. In diesem Zusammenhang ist auch eine Co-Medikation mit anderen Therapeutika bzw. Arzneimitteln, insbesondere Kortikosteroiden möglich.

Gleichermaßen kann die erfindungsgemäße monoterpenhaltige Darreichungsform auch zur prophylaktischen und/oder therapeutischen Behandlung bzw. zur Herstellung eines Arzneimittels zur prophylaktischen und/oder therapeutischen Behandlung von entzündlichen Erkrankungen der Gallenwege, insbesondere Cholezystitis und Cholangitis, angewendet werden.

Des Weiteren kann die monoterpenhaltige Darreichungsform nach der vorliegenden Erfindung auch zur prophylaktischen und/oder therapeutischen Behandlung bzw. zur Herstellung eines Arzneimittels zur prophylaktischen und/oder therapeutischen Behandlung von entzündlichen Erkrankungen der ableitenden Harnwege, insbesondere Glomerulonephritis, Pyelonephritis, Zystitis und Uritritis, Anwendung finden.

Ebenfalls kann die erfindungsgemäße monoterpenhaltige Darreichungsform zur prophylaktischen und/oder therapeutischen Behandlung bzw. zur Herstellung eines Arzneimittels zur prophylaktischen und/oder therapeutischen Behandlung von entzündlichen Erkrankungen des Darms, insbesondere Morbus Crohn und Colitis ulcerosa, verwendet werden.

Darüber hinaus ist es auch möglich, dass die erfindungsgemäße monoterpenhaltige Darreichungsform Anwendung findet bei der prophylaktischen und/oder therapeutischen Behandlung bzw. zur Herstellung eines Arzneimittels zur prophylaktischen und/oder therapeutischen Behandlung von entzündlichen oder allergischen Hauterkrankungen, insbesondere Ekzemen und Dermatitis.

Gleichermaßen kann die erfindungsgemäße monoterpenhaltige Darreichungsform zur prophylaktischen und/oder therapeutischen Behandlung bzw. zur Herstellung eines Arzneimittels zur prophylaktischen und/oder therapeutischen Behandlung von rheumatoiden Erkrankungen, insbesondere Rheuma und/oder Rheumatismus sowie Erkrankungen des rheumatischen Formenkreises, angewendet werden.

Schließlich kann die erfindungsgemäße monoterpenhaltige Darreichungsform auch Anwendung finden zur prophylaktischen und/oder therapeutischen Behandlung bzw. zur Herstellung eines Arzneimittels zur prophylaktischen und/oder therapeutischen Behandlung von systemisch kortikosteroidpflichtigen Erkrankungen, insbesondere Erkrankungen, welche mit systemisch verabreichten Kortikosteroiden therapiert werden. Dies gilt insbesondere bei Verwendung von 1,8-Cineol als monoterpenhaltige Wirksubstanz, da 1,8-Cineol ein kortikosteroidartiges pharmakologisches Wirkprofil aufweist.

Bei allen vorstehenden Verwendungen ist eine Co-Medikation mit mindestens einem weiteren Therapeutikum und/oder Arzneimittel möglich.

Im Rahmen der Verwendung wird die monoterpenhaltige Wirksubstanz, insbesondere 1,8-Cineol, in Tagesdosen von 50 bis 3.000 mg/diem, insbesondere 100 bis 2.000 mg/diem, vorzugsweise 200 bis 1.000 mg/diem, verabreicht bzw. wird die monoterpenhaltige Wirksubstanz, insbesondere 1,8-Cineol, zur Verabreichung in einer Tagesdosis von 50 bis 3.000 mg/diem, insbesondere 100 bis 2.000 mg/diem, vorzugsweise 200 bis 1.000 mg/diem, hergerichtet.

Schließlich ist Gegenstand der vorliegenden Erfindung - gemäß einem **dritten** Aspekt der vorliegenden Erfindung - ein Arzneimittel oder Medizinprodukt, welches eine monoterpenhaltige, insbesondere cineolhaltige, Darreichungsform umfasst, wie sie zuvor beschrieben worden ist.

Im Rahmen des erfindungsgemäßen Arzneimittels oder Medizinprodukt ist das Arzneimittel oder Medizinprodukt insbesondere für eine Verabreichung von monoterpenhaltiger Wirksubstanz, insbesondere 1,8-Cineol, in einer Tagesdosis von 50 bis 3.000 mg/diem, insbesondere 100 bis 2.000 mg/diem, vorzugsweise 200 bis 1.000 mg/diem, hergerichtet.

Für weitergehende diesbezügliche Einzelheiten zu dem erfindungsgemäßen Arzneimittel oder Medizinprodukt kann zur Vermeidung unnötiger Wiederholungen auf die obigen Ausführungen zu der erfindungsgemäßen Darreichungsform und dem erfindungsgemäßen Herstellungsverfahren Bezug genommen werden, welche hinsichtlich des erfindungsgemäßen Arzneimittels oder Medizinprodukts entsprechend gelten.

Die vorliegende Erfindung wird anhand der folgenden Ausführungsbeispiele veranschaulicht, welche die vorliegende Erfindung jedoch keinesfalls beschränken.

### Ausführungsbeispiele:

### 1. Herstellungsbeispiele:

### Beispiel A:

Eine erfindungsgemäße Darreichungsform in Form einer Kapsel mit Kern/Hülle-Struktur wird wie folgt hergestellt: Zunächst wird eine homogene Mischung auf Basis von 1,8-Cineol einerseits und mittelkettigen Triglyceriden andererseits in einem Gewichtsverhältnis von etwa 2 : 1 hergestellt und nach dem sogenannten *Rotary-Die-*Verfahren (synonym auch als "Scherer-Verfahren" bezeichnet) in dem Fachmann an sich bekannter Weise in Weichgelatinekapseln eingeschlossen bzw. zu Weichgelatinekapseln verarbeitet. Das Gemisch aus 1,8-Cineol und mittelkettigen Triglyceriden bildet den Kapselkern der erfindungsgemäßen Darreichungsform, und die Weichgelatine stellt die erste Kapselhülle dar. Der Kapselkern hat ein Gewicht von ca. 150 mg. Das Material der ersten Kapselhülle enthält neben Gelatine etwa 12 Gew.-% Sorbitol sowie etwa 15 Gew.-% Glycerin.

Die so erhaltenen Weichgelatinekapseln werden anschließend in einem sogenannten *Pan-Coater* mittels Sprühauftrag mit einer zweiten Kapselhülle versehen. Die Kapseln werden zu diesem Zweck mit einer wässrigen Dispersion von Ethylcellulose und Natriumalginat, deren einzelne Bestandteile kommerziell erhältlich sind, in einem gewichtsbezogenen Verhältnis Ethylcellulose/ Natriumalginat von 6 : 1 bei Raumtemperatur besprüht. Die Dispersion wird zuvor mit Ammoniak basisch eingestellt und weist zudem einen Feststoffanteil von ca. 10 Gew.-% auf. Ferner enthält die Dispersion neben Ethylcellulose und Natriumalginat noch geringe Mengen an Ölsäure, Stearinsäure und mittelkettigen Triglyceriden. Für die Beschichtung von 1 Million Kapseln werden ca. 10 Stunden benötigt, um Kapseln mit einer stabilen zweiten Kapselhülle der gewünschten Schichtdicke zu erhalten.

In einem abschließenden Verfahrensschritt wird die Kapseloberfläche mit geringen Mengen an Candelillawachs behandelt. Es resultiert eine erfindungsgemäße Darreichungsform in Form von Kern/Hülle-Kapseln mit dem zuvor beschriebenen Aufbau.

### Beispiel B:

Beispiel B wird analog zu Beispiel A durchgeführt, wobei jedoch der Kapselkern ein Gewicht von etwa 300 mg aufweist.

### Beispiele C bis F:

Weiterhin werden vier weitere erfindungsgemäße Darreichungsformen entsprechend Beispiel A hergestellt, wobei jedoch das Mischungsverhältnis von 1,8-Cineol zu Triglyceriden im Bereich von 10 : 1 bis 1 : 5 variiert wird und darüber hinaus bei einer Darreichungsform (Beispiel E) 1,8-Cineol als Reinsubstanz eingesetzt wird. Die genauen Mischungsverhältnisse sind in Tabelle 1 wiedergegeben.

### Beispiele G bis J:

Des Weiteren werden vier wiederum weitere Herstellungsbeispiele für erfindungsgemäße Darreichungsformen entsprechend Beispiel A durchgeführt, bei welchen jedoch das Verhältnis von Natriumalginat zu Ethylcellulose in der zweiten Kapselhülle im Bereich von 1 : 2 bis 4 : 1 variiert wird. Die genauen Verhältnisse sind in Tabelle 1 wiedergegeben.

### Beispiele K und L:

Schließlich werden noch zwei weitere erfindungsgemäße Herstellungsbeispiele für erfindungsgemäße Darreichungsformen entsprechend Beispiel A hergestellt, wobei jedoch in Beispiel K die zweite Kapselhülle weder Stearinsäure noch Fettsäure oder Triglyceride aufweist. Beispiel L entspricht Beispiel A, wobei jedoch auf die abschließende Beschichtung bzw. Behandlung mit Candelillawachs verzichtet wird.

### Beispiele M bis O (vergleich):

In Anlehnung an Herstellungsbeispiel A werden drei Vergleichsbeispiele hergestellt, bei welchen jedoch jeweils das Material der zweiten Kapselhülle abweichend von der erfindungsgemäßen Darreichungsform variiert wird.

In Beispiel M wird eine Beschichtung aus Polymethacrylat, welche unter der Handelsbezeichnung Eudragit^{®} erhältlich ist, verwendet. Zur Aufbringung dieser Beschichtung wird die entsprechende Dispersion bei 80 °C auf die Kapseln gesprüht, wobei für die Beschichtung von 1 Million Kapseln eine Zeit von 15 Stunden benötigt wird. Für die Herstellung wird eine apparativ aufwendige Herstellungseinheit mit Beheizungssystem und Homogenisierungsvorrichtungen benötigt.

Herstellungsbeispiel N entspricht Beispiel A, wobei jedoch das Material der zweiten Kapselhülle kein Alginat enthält.

Herstellungsbeispiel O entspricht Herstellungsbeispiel A, wobei jedoch das Material der zweiten Kapselhülle keine Ethylcellulose aufweist.

### 2. Untersuchungen und Anwendungsbeobachtungen:

Die gemäß den Beispielen A bis O hergestellten Kapseln werden hinsichtlich ihrer haptischen und optischen Eigenschaften (Oberflächenglätte und Glanz), des Ablöseverhaltens der zweiten Kapselhülle von der ersten Kapselhülle sowie ihres Auflöseverhaltens in künstlichem Magensaft bzw. künstlichem Darmsaft untersucht und bewertet. Des Weiteren wurde die mechanische Belastbarkeit mittels Druckbeaufschlagung ("Eindrückversuch") bei definierter Druckeinwirkung untersucht. Die entsprechenden Ergebnisse sind in Tabelle 1 zusammengefasst.

Die Magensaftresistenz bzw. die Dünndarmlöslichkeit der Kapseln werden nach den Standards gemäß European Pharmacopoeia 7.0, 04/2010: 1502, Seiten 707 bis 709, Stichwort "Capsules", Unterkapitel "Gastro-Resistant Capsules", sowie European Pharmacopoeia 7.1, 04/2011: 20901, Seiten 3331 und 3332, Stichwort "2.9.1 Disintergration of Tablets and Capsules", durchgeführt. Die Kapseln müssen eine Behandlung von mindestens 2 Stunden in einer künstlichen Magensaftflüssigkeit, welche aus 0,1 N Salzsäure besteht, bei Temperaturen im Bereich von 35 bis 39 °C und unter ständigem Rühren so unbeschadet überstehen, dass kein Wirkstoff freigesetzt wird, um als magensaftresistent zu gelten.

Zum Test der Dünndarmlöslichkeit wurden die Kapseln anschließend in eine Phosphatpufferlösung, welche auf einen pH-Wert von 6,8 eingestellt ist, unter Rühren bei Temperaturen im Bereich von 35 bis 39 °C gerührt. Bei diesem Test mussten die Kapseln innerhalb einer Stunde mindestens soweit zersetzt sein, dass der Kapselinhalt freigesetzt wird.

Die Magensaftresistenz und die Dünndarmlöslichkeit werden qualitativ mittels einer Bewertungsskala von "+" bis "+++" bewertet: "+++" entspricht einer vollständigen Resistenz gegenüber dem künstlichen Magensaft bzw. einer kontrollierten, retardierten Freisetzung der Wirkstoffe in künstlichen Dünndarmsaft. "++" entspricht einem Anlösen bzw. einer oberflächlichen Beschädigung der Kapsel in künstlichem Magensaft, wobei jedoch kein Wirkstoff freigesetzt wird, bzw. einer vollständigen Freisetzung des Wirkstoffs in künstlichem Dünndarmsaft, wobei die Dauer der Freisetzung nicht mehr exakt gesteuert werden kann. "+" entspricht einem Anlösen bzw. einer Beschädigung der Kapsel in künstlichem Magensaft, wobei es auch zu einer Freisetzung des Wirkstoffs kommen kann, bzw. einer kaum noch kontrollierbaren bzw. unvollständigen Freisetzung des Wirkstoffs in künstlichem Darmsaft.

Die Oberflächenglätte, der Glanz und das Ablöseverhalten werden nach einem Schulnotensystem von "1" bis "6" beurteilt, wobei die Note "1" sehr gut und die Note "6" ungenügend entspricht.

Wie sich aus den Daten in Tabelle 1 ersehen lässt, weisen die Kapseln nach Beispiel A die besten Eigenschaften auf - sowohl hinsichtlich ihres Auflöseverhaltens als auch hinsichtlich der optischen und mechanischen Eigenschaften.

Die Werte für Beispiel B sind innerhalb der Messungenauigkeiten mit denen von Beispiel A identisch, was zeigt, dass eine Variation der Größe des Kapselkerns keinen bzw. keinen bedeutenden Einfluss auf das Auflöseverhalten bzw. die mechanischen, optischen und haptischen Eigenschaften der Kapseln besitzt.

Überraschenderweise zeigt sich jedoch, dass die Variation des Verhältnisses von monoterpenhaltiger Wirksubstanz zu Trägermaterial bzw. Exzipienten im Kapselkern sowohl das Auflöseverhalten als auch die optischen und haptischen Eigenschaften der Kapseln beeinflussen kann (vgl. Beispiele C bis F in Tabelle 1). Die Beispiele C bis F weisen gegenüber Beispiel A leicht verschlechterte Eigenschaften auf, sind jedoch immer noch deutlich leistungsstärker als die Vergleichsbeispiele M bis O.

Die Werte in Tabelle 1 belegen ferner, dass eine Variation von Alginat zu Ethylcellulose in der zweiten Kapselhülle erwartungsgemäß sowohl das Auflöseverhalten der Kapseln beeinflusst, darüber hinaus jedoch auch die mechanischen, haptischen und optischen Eigenschaften der Kapseln nicht unwesentlich verändert.

Ferner zeigen auch die Beispiele K und L, dass das Auflöseverhalten und die Anwendungseigenschaften, insbesondere die mechanischen, haptischen und optischen Eigenschaften, der erfindungsgemäßen Kapseln durch die Anwesenheit von Hilfsstoffen, insbesondere von Feuchthaltemitteln und Weichmachern, in der zweiten Kapselhülle sowie eine abschließende Behandlung der Kapseln mit einem Wachs beträchtlich verbessert werden können.

**Tabelle 1: Zusammensetzung und Eigenschaften der Kapseln gemäß den Beispielen A bis O**

| Beispiel | Kapselkern [mg] | Cineol/ Triglycerid-Verhältnis **^{a)}** | Gewichtsverhältnis Alginat/Ethylcellulose in zweiter Kapselhülle | Magensaftresistenz | Dünndarm-löslichkeit | Oberflächen-glätte | Glanz | Ablöseverhalten | Mechanische Belastbarkeit |
|---|---|---|---|---|---|---|---|---|---|
| A | 150 | 2 : 1 | 1 : 6 | +++ | +++ | 1 | 1 | 1 | 1 |
| B | 300 | 2 : 1 | 1 : 6 | +++ | +++ | 1 | 1 | 1 | 1 |
| C | 150 | 4 : 1 | 1 : 6 | +++ | ++ | 1-2 | 1 | 1-2 | 1 |
| D | 150 | 10 : 1 | 1 : 6 | ++ | ++ | 1-2 | 1 | 2 | 1-2 |
| E | 150 | 1,8-Cineol **^{b)}** | 1 : 6 | + | ++ | 2 | 1-2 | 2 | 2 |
| F | 150 | 1 : 5 | 1 : 6 | +++ | ++ | 1-2 | 1-2 | 1 | 1-2 |
| G | 150 | 2 : 1 | 1 : 2 | +++ | +++ | 2 | 1-2 | 1-2 | 1 |
| H | 150 | 2 : 1 | 1 : 1 | +++ | ++ | 2 | 1-2 | 2 | 2 |
| I | 150 | 2 : 1 | 2 : 1 | +++ | ++ | 1-2 | 2 | 2 | 2-3 |
| J | 150 | 2 : 1 | 4 : 1 | +++ | ++ | 1-2 | 2 | 2-3 | 2-3 |
| K | 150 | 2 : 1 | 1 : 6 | +++ | ++ | 1 | 1 | 1-2 | 1-2 |
| L | 150 | 2 : 1 | 1 : 6 | +++ | +++ | 1-2 | 2 | 1 | 1 |
| M | 150 | 2 : 1 | Eudragit^{® **d)**} | +++ | ++ | 2-3 | 2-3 | 3-4 | 3 |
| N | 150 | 2 : 1 | Ethylcellulose **^{d)}** | +++ | c) | 2 | 2 | 4-5 | 1-2 |
| O | 150 | 2 : 1 | Alginat**^{d)}** | +++ | + | 2 | 1-2 | 4 | 4 |

| | | | | | | | | | |
|---|---|---|---|---|---|---|---|---|---|
| a) Gewichtsverhältnis von 1,8-Cineol zu mittelkettigen Triglyceriden im Kapselkern b) 1,8-Cineol als Reinsubstanz im Kapselkern c) nicht löslich (Es wird sporadisch ein Quellen und Reißen der Kapsel beobachtet, welches jedoch nicht reproduzierbar ist.) d) zweite Kapselhülle aus angegebenem Material | | | | | | | | | |

Des Weiteren werden mit den Kapseln gemäß den Herstellungsbeispielen A, D, J, K sowie mit den Kapseln gemäß Vergleichsbeispiel M Langzeitversuche durchgeführt, bei welchen die Kapseln nach drei, sechs und zwölf Monaten Lagerung nochmals untersucht werden. Die Kapseln werden dabei bei einer Temperatur von 40 °C bei einer relativen Luftfeuchte von 75 % (Test gemäß ICH-Standard) gelagert. Die Ergebnisse der Tests sind in Tabelle 2 wiedergegeben.

**Tabelle 2: Langzeitbeobachtungen ausgewählter Systeme**

| **Beispiele** | **A** | **D** | **J** | **K** | **M** |
|---|---|---|---|---|---|
| | | | | | |

| **3 Monate** | | | | | |
|---|---|---|---|---|---|
| Magensaftresistenz | +++ | ++ | +++ | +++ | +++ |
| Dünndarmlöslichkeit | +++ | ++ | ++ | ++ | ++ |
| Ablöseverhalten | 1 | 2 | 2-3 | 1-2 | 3-4 |
| | | | | | |

| **6 Monate** | | | | | |
|---|---|---|---|---|---|
| Magensaftresistenz | +++ | ++ | ++ | ++ | +++ |
| Dünndarmlöslichkeit | +++ | ++ | ++ | ++ | ++ |
| Ablöseverhalten | 1 | 2-3 | 3 | 2 | 4 |
| | | | | | |

| **12 Monate** | | | | | |
|---|---|---|---|---|---|
| Magensaftresistenz | +++ | ++ | ++ | ++ | ++ |
| Dünndarmlöslichkeit | +++ | ++ | + | ++ | ++ |
| Ablöseverhalten | 1 | 3 | 3 | 2 | 4 |

## Patentansprüche

1. Monoterpenhaltige Darreichungsform für die perorale Applikation in Form einer Kapsel, insbesondere in Form einer Kapsel mit Kern/Hülle-Struktur mit mehrschichtiger Hülle, wobei die Kapsel von innen nach außen den folgenden Aufbau aufweist:
(a) einen Kapselkern, enthaltend mindestens eine monoterpenhaltige Wirksubstanz, insbesondere mindestens ein Monoterpen,
(b) eine erste, den Kapselkern umgebende, insbesondere den Kapselkern unmittelbar umgebende, strukturgebende und/oder formgebende Kapselhülle und
(c) eine zweite, die erste Kapselhülle umgebende, insbesondere die erste Kapselhülle unmittelbar umgebende, Kapselhülle, vorzugsweise in Form einer Beschichtung, insbesondere in Form einer magensaftresistenten, aber dünndarmlöslichen Beschichtung, wobei die zweite Kapselhülle mindestens zwei Komponenten (Inhaltsstoffe bzw. Bestandteile) umfasst, wobei eine erste Komponente ("Komponente 1") auf Basis von Alginsäure oder deren physiologisch verträglichen Salzen oder Estern und eine zweite Komponente ("Komponente 2") auf Cellulosebasis ausgebildet ist, wobei die Darreichungsform einen gewichtsbezogenen Anteil der zweiten Kapselhülle, bezogen die Darreichungsform, im Bereich von 0,5 bis 4 Gew.-% aufweist.

2. Darreichungsform nach Anspruch 1,
wobei die monoterpenhaltige Wirksubstanz, insbesondere das Monoterpen, ausgewählt ist aus der Gruppe von 1,8-Cineol, Menthol, Campher, Limonen, Carvon, Carveol oder deren Kombinationen, insbesondere wobei das Monoterpen 1,8-Cineol und/oder Menthol, besonders bevorzugt 1,8-Cineol, ist; und/oder
wobei die Darreichungsform, insbesondere der Kapselkern, 1,8-Cineol als alleinigen Wirkstoff enthält oder aber wobei die Darreichungsform, insbesondere der Kapselkern, 1,8-Cineol mit mindestens einem weiteren Wirkstoff, bevorzugt ausgewählt aus Terpenen, enthält.

3. Darreichungsform nach einem der vorangehenden Ansprüche, **dadurch gekennzeichnet, dass** die Darreichungsform, insbesondere der Kapselkern, die monoterpenhaltige Wirksubstanz und/oder das Monoterpen, insbesondere 1,8-Cineol, zusammen mit mindestens einem mit der monoterpenhaltigen Wirksubstanz und/oder dem Monoterpen, insbesondere 1,8-Cineol, mischbaren und/oder hierin löslichen, insbesondere bei 20 °C und Atmosphärendruck flüssigen, physiologisch unbedenklichen Träger (Exzipienten) enthält;
insbesondere wobei der Träger ausgewählt ist aus der Gruppe von fetten Ölen, bevorzugt Triglyceriden, besonders bevorzugt mittelkettigen Triglyceriden *(Medium Chain Triglycerides,* MCT), ganz besonders bevorzugt Triglyceriden mit C₆-C₁₂-Fettsäureresten; und/oder
insbesondere wobei der Träger in einem monoterpenhaltige Wirksubstanz/Träger-Mengenverhältnis im Bereich von 1.000:1 bis 1:1.000, insbesondere 100:1 bis 1 : 100, vorzugsweise 50 : 1 bis 1 : 50, besonders bevorzugt 10:1 bis 1:10, ganz besonders bevorzugt 5 : 1 bis 1 : 2, noch mehr bevorzugt 3 : 1 bis 1:1, eingesetzt wird.

4. Darreichungsform nach einem der vorangehenden Ansprüche,
wobei die Darreichungsform die monoterpenhaltige Wirksubstanz in absoluten Mengen von 10 bis 1.000 mg, insbesondere 25 bis 750 mg, vorzugsweise 50 bis 500 mg, besonders bevorzugt 75 bis 300 mg, enthält, insbesondere bezogen auf eine Applikationseinheit, insbesondere Kapsel; und/oder
wobei die Darreichungsform die monoterpenhaltige Wirksubstanz in relativen Mengen von 0,01 bis 99 Gew.-%, insbesondere 0,1 bis 95 Gew.-%, vorzugsweise 1 bis 90 Gew.-%, besonders bevorzugt 5 bis 80 Gew.-%, bezogen auf die Darreichungsform, enthält; und/oder
wobei die Darreichungsform den Kapselkern in Mengen von 10 bis 1.500 mg, insbesondere 50 bis 1.250 mg, vorzugsweise 75 bis 1.000 mg, bevorzugt 100 bis 750 mg, besonders bevorzugt 125 bis 500 mg, enthält und/oder wobei die Darreichungsform einen gewichtsbezogenen Anteil des Kapselkerns, bezogen die Darreichungsform, im Bereich von 10 bis 90 Gew.-%, insbesondere 20 bis 85 Gew.-%, vorzugsweise 40 bis 80 Gew.-%, bevorzugt 50 bis 75 Gew.-%, besonders bevorzugt 60 bis 70 Gew.-%, aufweist,; und/oder
wobei die erste Kapselhülle als Weichkapsel und/oder Weichkapselhülle ausgebildet ist und/oder wobei das Kapselhüllenmaterial der ersten Kapselhülle mindestens ein pharmakologisch unbedenkliches Polymer umfasst oder hieraus besteht, insbesondere ausgewählt aus synthetischen, natürlichen oder naturidentischen Polymeren und Polymerisaten, bevorzugt ausgewählt aus der Gruppe von Proteinen, vorzugsweise Gelatine und/oder Caseinaten, bevorzugt Gelatine, sowie deren Mischungen und Kombinationen; und/oder
wobei das Kapselhüllenmaterial der ersten Kapselhülle Gelatine, insbesondere Hart- oder Weichgelatine, vorzugsweise Weichgelatine, umfasst oder hieraus besteht, gegebenenfalls zusammen mit mindestens einem weiteren Additiv oder Hilfsstoff, insbesondere ausgewählt aus Weichmachern, Feuchthaltemitteln und Schmiermitteln sowie deren Mischungen und Kombinationen, insbesondere wobei die Menge an Additiv oder Hilfsstoff insgesamt 0,01 bis 40 Gew.-%, insbesondere 0,1 bis 35 Gew.-%, vorzugsweise 1 bis 30 Gew.-%, bezogen auf die erste Kapselhülle, beträgt; und/oder wobei die Darreichungsform die erste Kapselhülle in Mengen von 40 bis 500 mg,
insbesondere 50 bis 400 mg, vorzugsweise 60 bis 300 mg, bevorzugt 70 bis 250 mg, besonders bevorzugt 80 bis 200 mg, enthält und/oder wobei die Darreichungsform einen gewichtsbezogenen Anteil der ersten Kapselhülle, bezogen die Darreichungsform, im Bereich von 15 bis 75 Gew.-%, insbesondere 20 bis 65 Gew.-%, vorzugsweise 25 bis 55 Gew.-%, bevorzugt 30 bis 50 Gew.-%, besonders bevorzugt 35 bis 45 Gew.-%, aufweist.

5. Darreichungsform nach einem der vorangehenden Ansprüche,
wobei die zweite Kapselhülle als erste Komponente mindestens ein Alginat aufweist, insbesondere ausgewählt aus Alkalimetallalginaten, Erdalkalimetallalginaten, Ammoniumalginaten und Alginsäureestern, vorzugsweise ausgewählt aus Natriumalginat, Kaliumalginat, Ammoniumalginat, Calciumalginat und Propylenglykolalginat, bevorzugt ausgewählt aus Natriumalginat, Kaliumalginat, Ammoniumalginat und Calciumalginat, besonders bevorzugt Natriumalginat; und/oder wobei die zweite Kapselhülle als zweite Komponente Cellulose oder ein Cellulosederivat,
insbesondere einen Celluloseether oder Celluloseester, enthält, insbesondere wobei die zweite Komponente ausgewählt ist aus Celluloseethern, vorzugsweise aus der Gruppe von Methylcellulose, Ethylcellulose, Ethylmethylcellulose, Hydroxypropylcellulose, Hydroxypropylmethylcellulose, Carboxymethylcellulose oder deren Kombinationen, vorzugsweise Ethylcellulose; und/oder
wobei die zweite Kapselhülle die erste und die zweite Komponente in gewichtsbezogenen Verhältnissen von erster Komponente zu zweiter Komponente ("Komponente 1/Komponente 2-Gewichtsverhältnis") von 1 : 99 bis 50 : 50, insbesondere 5 : 95 bis 40 : 60, vorzugsweise 8 : 92 bis 30 : 70, bevorzugt 10 : 90 bis 25 : 75, besonders bevorzugt 12 : 88 bis 20 : 80, aufweist; und/oder wobei die zweite Kapselhülle außerdem mindestens einen weiteren Bestandteil oder
Inhaltsstoff, insbesondere ausgewählt aus Additiven oder Hilfsstoffen, vorzugsweise aus fetten Ölen und/oder Fettsäuren, besonders bevorzugt ausgewählt aus der Gruppe von Stearinsäure, Ölsäure und Triglyceriden sowie deren Mischungen und Kombinationen, aufweist, insbesondere wobei die Triglyceride aus mittelkettigen Triglyceriden (*Medium Chain Triglycerides,* MCT), vorzugsweise aus Triglyceriden mit C₆-C₁₂-Fettsäureresten, ausgewählt sind und/oder insbesondere wobei die Menge an weiterem Bestandteil oder Inhaltsstoff insgesamt 0,01 bis 35 Gew.-%, insbesondere 0,1 bis 30 Gew.-%, vorzugsweise 1 bis 25 Gew.-%, bezogen auf die zweite Kapselhülle, betragen kann; und/oder
wobei die zweite Kapselhülle basisch eingestellt ist.

6. Darreichungsform nach einem der vorangehenden Ansprüche,
wobei die Darreichungsform die zweite Kapselhülle in Mengen von 0,5 bis 100 mg, insbesondere 1 bis 75 mg, vorzugsweise 1 bis 50 mg, bevorzugt 2 bis 40 mg, besonders bevorzugt 3 bis 35 mg, ganz besonders bevorzugt 5 bis 30 mg, enthält und/oder wobei die Darreichungsform einen gewichtsbezogenen Anteil der zweiten Kapselhülle, bezogen die Darreichungsform, im Bereich von 2 bis 4 Gew.-% aufweist; und/oder
wobei der Durchmesser, insbesondere der mittlere Durchmesser, der Darreichungsform im Bereich von 50 µm bis 25 mm, insbesondere im Bereich von 100µm bis 25 mm, vorzugsweise im Bereich von 250 µm bis 25 mm, besonders bevorzugt im Bereich von 500 µm bis 25 mm, ganz besonders bevorzugt im Bereich von 750 µm bis 25 mm, noch mehr bevorzugt im Bereich von 1.000 µm bis 25 mm, variiert; und/oder
wobei das Verhältnis der Dicke der ersten Kapselhülle zur Dicke der zweiten Kapselhülle 500 : 1 bis 1 : 1, insbesondere 100 : 1 bis 2 : 1, vorzugsweise 50 : 1 bis 5 : 1, beträgt; und/oder
wobei das gewichtsbezogene Verhältnis von erster Kapselhülle zu zweiter Kapselhülle 1 : 1 bis 50 : 1, insbesondere 2 : 1 bis 30 : 1, vorzugsweise 3 : 1 bis 25 : 1, bevorzugt 5 : 1 bis 20 : 1, beträgt.

7. Darreichungsform nach einem der vorangehenden Ansprüche, wobei die Darreichungsform mit einer äußeren, die zweite Kapselhülle umgebenden, insbesondere die zweite Kapselhülle unmittelbar umgebenden, Beschichtung, insbesondere auf Basis von Lacken oder Wachsen sowie deren Mischungen und Kombinationen, versehen ist.

8. Darreichungsform nach einem der vorangehenden Ansprüche
zur Verwendung in der Humanmedizin oder Veterinärmedizin und/oder zur Verwendung bei der prophylaktischen und/oder therapeutischen Behandlung des menschlichen oder tierischen Körpers; oder
zur Verwendung bei der prophylaktischen und/oder therapeutischen Behandlung von entzündlichen, infektexazerbierten oder allergischen Erkrankungen des menschlichen oder tierischen Körpers; oder
zur Verwendung bei der prophylaktischen und/oder therapeutischen Behandlung von Autoimmunerkrankungen des menschlichen oder tierischen Körpers; oder
zur Verwendung bei der prophylaktischen und/oder therapeutischen Behandlung von Erkältungserkrankungen und grippalen Infekten und hiermit zusammenhängenden Erkrankungen und Infekten, insbesondere Infekten der oberen und unteren Atemwege, insbesondere Rhinitis, Sinusitis und bronchopulmonalen Erkrankungen; oder
zur Verwendung bei der prophylaktischen und/oder therapeutischen Behandlung von Atemwegserkrankungen, insbesondere von bronchopulmonalen Erkrankungen, insbesondere Bronchitis, Asthma bronchiale und chronischen obstruktiven Lungenerkrankungen (COPD); oder
zur Verwendung bei der prophylaktischen und/oder therapeutischen Behandlung von entzündlichen Erkrankungen der Gallenwege, insbesondere Cholezystitis und Cholangitis; oder
zur Verwendung bei der prophylaktischen und/oder therapeutischen Behandlung von entzündlichen Erkrankungen der ableitenden Harnwege, insbesondere Glomerulonephritis, Pyelonephritis, Zystitis und Uritritis; oder
zur Verwendung bei der prophylaktischen und/oder therapeutischen Behandlung von entzündlichen Erkrankungen des Darms, insbesondere Morbus Crohn und Colitis ulcerosa; oder
zur Verwendung bei der prophylaktischen und/oder therapeutischen Behandlung von entzündlichen oder allergischen Hauterkrankungen, insbesondere Ekzemen und Dermatitis; oder
zur Verwendung bei der prophylaktischen und/oder therapeutischen Behandlung von rheumatoiden Erkrankungen, insbesondere Rheuma und/oder Rheumatismus sowie Erkrankungen des rheumatischen Formenkreises; oder
zur Verwendung bei der prophylaktischen und/oder therapeutischen Behandlung von systemisch kortikosteroidpflichtigen Erkrankungen, insbesondere Erkrankungen, welche mit systemisch verabreichten Kortikosteroiden therapiert werden.

9. Darreichungsform nach Anspruch 8, wobei die monoterpenhaltige Wirksubstanz, insbesondere 1,8-Cineol, in Tagesdosen von 50 bis 3.000 mg/diem, insbesondere 100 bis 2.000 mg/diem, vorzugsweise 200 bis 1.000 mg/diem, verabreicht wird und/oder wobei die monoterpenhaltige Wirksubstanz, insbesondere 1,8-Cineol, zur Verabreichung in einer Tagesdosis von 50 bis 3.000 mg/diem, insbesondere 100 bis 2.000 mg/diem, vorzugsweise 200 bis 1.000 mg/diem, hergerichtet ist.

10. Arzneimittel oder Medizinprodukt, umfassend eine monoterpenhaltige, insbesondere cineolhaltige, Darreichungsform nach einem der vorangehenden Ansprüche.

11. Verfahren zur Herstellung einer monoterpenhaltigem Darreichungsform für die perorale Applikation in Form einer Kapsel, insbesondere in Form einer Kapsel mit Kern/Hülle-Struktur mit mehrschichtiger Hülle, wie einem der vorangehenden Ansprüche definiert, wobei
(i) in einem ersten Verfahrensschritt ein Kapselkern, enthaltend mindestens eine monoterpenhaltige Wirksubstanz, insbesondere mindestens ein Monoterpen, mit einer ersten, den Kapselkern umgebenden, insbesondere den Kapselkern unmittelbar umgebenden, strukturgebenden und/oder formgebenden Kapselhülle versehen wird und
(ii) in einem nachfolgenden zweiten Verfahrensschritt die erste Kapselhülle mit einer zweiten, die erste Kapselhülle umgebenden, insbesondere die erste Kapselhülle unmittelbar umgebenden, Kapselhülle, vorzugsweise in Form einer Beschichtung, insbesondere in Form einer magensaftresistenten, aber dünndarmlöslichen Beschichtung, versehen wird, wobei zur Herstellung der zweiten Kapselhülle mindestens zwei Komponenten eingesetzt werden, wobei eine erste Komponente ("Komponente 1") auf Basis von Alginsäure oder deren physiologisch verträglichen Salzen oder Estern und eine zweite Komponente ("Komponente 2") auf Cellulosebasis ausgebildet ist, wobei die erste und die zweite Komponente zur Herstellung der zweiten Kapselhülle gemeinsam in Form einer wässrigen Lösung und/oder Dispersion aufgebracht werden, wobei die Lösung und/oder Dispersion basisch eingestellt wird und wobei die erhaltene Darreichungsform einen gewichtsbezogenen Anteil der zweiten Kapselhülle, bezogen die Darreichungsform, im Bereich von 0,5 bis 4 Gew.-% aufweist.

12. Verfahren nach Anspruch 11,
wobei die Lösung und/oder Dispersion mittels wässriger Ammoniaklösung basisch eingestellt wird; und/oder
wobei die Lösung und/oder Dispersion bei Temperaturen im Bereich von 10 bis 70 °C, insbesondere 15 bis 60 °C, vorzugsweise 20 bis 50 °C, bevorzugt 25 bis 40 °C, aufgebracht wird;
wobei die Lösung und/oder Dispersion mittels Tauch- und/oder Sprühverfahren, insbesondere mittels Sprühverfahren, aufgebracht wird; und/oder
wobei in einem abschließenden Schritt (iii) mindestens eine Beschichtung, insbesondere auf Basis von Lacken oder Wachsen sowie deren Mischungen und Kombinationen, auf die zweite Kapselhülle aufgebracht wird, insbesondere unmittelbar auf die zweite Kapselhülle aufgebracht wird.

## Claims

1. Monoterpene-containing dosage form for peroral administration in the form of a capsule, especially in the form of a capsule having a core/shell structure with multi-layer envelope, wherein the said capsule has the following structure from the inside to the outside:
(a) a capsule core, comprising at least one monoterpene-containing active substance, in particular at least a monoterpene,
(b) a first capsule shell surrounding the capsule core, especially directly surrounding the capsule core, in order to structure and/or form it, and
(c) a second capsule shell surrounding the first capsule shell, especially directly surrounding the first capsule shell, preferably in the form of a coating, in particular in the form of a gastro-resistant but thin enteric coating, wherein the second capsule shell comprises at least two components (ingredients or components), wherein a first component ("component 1") is based on alginic acid or physiologically compatible salts or esters and a second component ("component 2") is based on cellulose, wherein the dosage form has a weight-based proportion with respect to the second capsule shell, depending on the dosage form, in the range of 0.5 to 4 wt.-%.

2. Dosage form according to claim 1,
wherein the said monoterpene-containing active substance, in particular the monoterpene, is selected from the group consisting of 1,8-cineole, menthol, camphor, limonene, carvone, carveol, or combinations thereof, in particular wherein the monoterpene is 1,8-cineole and/or menthol, particularly preferably 1,8-cineole; and/or
wherein the dosage form, especially the capsule core, contains 1,8-cineol as the sole active substance, or wherein the dosage form, especially the capsule core, contains 1,8-cineole with at least one other active substance, preferably selected from terpenes.

3. Dosage form according to one of the preceding claims, **characterised in that** the dosage form, especially the capsule core, contains the monoterpene-containing active substance and/or the monoterpene, in particular 1,8-cineole, together with at least a carrier (excipient) that is liquid and physiologically acceptable, in particular at 20°C and at atmospheric pressure, and is miscible and/or soluble therein, together with the monoterpene-containing active substance and/or the monoterpene, in particular 1,8-cineole;
in particular wherein the carrier is selected from the group of fatty oils, preferably triglycerides, more preferably Medium Chain Triglycerides (MCT), most preferably triglycerides with C₆-C₁₂ fatty acid esters; and/or
in particular wherein the carrier is used in a monoterpene-containing active substance/carrier proportion in the range of 1,000:1 to 1:1,000, in particular 100:1 to 1:100, preferably 50:1 to 1:50, more preferably 10:1 to 1:10, even more preferably 5:1 to 1:2, most preferably 3:1 to 1:1.

4. Dosage form according to any one of the preceding claims,
wherein the dosage form contains the monoterpene-containing active substance in absolute amounts of 10 to 1,000 mg, in particular 25 to 750 mg, preferably 50 to 500 mg, particularly preferably 75 to 300 mg, especially with respect to an application unit, in particular a capsule; and/or
wherein the dosage form contains the monoterpene-containing active substance in relative amounts of from 0.01 to 99 wt.-%, especially 0.1 to 95 wt.-%, preferably 1 to 90 wt.%, particularly preferably 5 to 80 wt.-%, with respect to the dosage form; and/or
wherein the dosage form contains the capsule core in amounts from 10 to 1,500 mg, in particular from 50 to 1,250 mg, preferably from 75 to 1,000 mg, more preferably from 100 to 750 mg, particularly preferably from 125 to 500 mg, and/or wherein the dosage form has a weight-related portion of the capsule core, depending on the dosage form, in the range from 10 to 90 wt.-%, in particular 20 to 85 wt.-%, preferably 40 to 80 wt.-%, more preferably 50 to 75 wt.-%, particularly preferably 60 to 70 wt.-%; and/or
wherein the first capsule shell is formed as a soft capsule and/or soft capsule shell, and/or wherein the capsule shell material of the first capsule shell includes or consists of at least a pharmacologically-acceptable polymer, in particular selected from synthetic, natural or nature-identical polymers and polymers preferably selected from the group of proteins, preferably gelatin and/or caseinates, preferably gelatin, as well as mixtures and combinations thereof; and/or
wherein the capsule shell material of the first capsule shell includes or consists of gelatin, in particular hard or soft gelatin, preferably soft gelatin, optionally together with at least a further additive or excipient, especially selected from emollients, humectants and lubricants as well as mixtures and combinations thereof, in particular wherein the total amount of additive or excipient is 0.01 to 40 wt.-%, especially 0.1 to 35 wt.-%, preferably 1 to 30 wt.-%, with respect to the first capsule shell; and/or
wherein the dosage form contains the first capsule shell in amounts of 40 to 500 mg, in particular 50 to 400 mg, preferably 60 to 300 mg, more preferably 70 to 250 mg, particularly preferably 80 to 200 mg, and/or wherein the dosage form has a weight-based proportion of the first capsule shell, depending on the dosage form, in the range of 15 to 75 wt.-%, in particular 20 to 65 wt.-%, preferably 25 to 55 wt.-%, preferably 30 to 50 wt.-%, particularly preferably 35 to 45 wt.-%.

5. Dosage form according to one of the preceding claims,
wherein the second capsule shell comprises, as the first component, at least an alginate, in particular selected from alkali metal alginate, alkaline earth metal alginate, ammonium alginate and alginic acid esters, preferably selected from sodium alginate, potassium alginate, ammonium alginate, calcium alginate and propylene glycol alginate, more preferably selected from sodium alginate, potassium alginate, ammonium alginate and calcium alginate, particularly preferably sodium alginate; and/or
wherein the second capsule shell comprises, as the second component, cellulose or a cellulose derivative, in particular a cellulose ether or cellulose ester, in particular wherein the second component is selected from cellulose ethers, preferably from the group of methylcellulose, ethylcellulose, ethylmethylcellulose, hydroxypropylcellulose, hydroxypropylmethylcellulose, carboxymethylcellulose or combinations thereof, more preferably ethyl cellulose; and/or
wherein the second capsule shell has, as the first and the second component in weight-based ratios of the first component to the second component ("component 1/component 2 weight ratio") of 1:99 to 50:50, in particular from 5:95 to 40:60, preferably 8:92 to 30:70, more preferably 10:90 to 25:75, particularly preferably 12:88 to 20:80,; and/or
wherein the second capsule shell further comprises at least a further ingredient or ingredients, in particular selected from additives or auxiliary agents, preferably from fatty oils and/or fatty acids, particularly preferably selected from the group consisting of stearic acid, oleic acid, and triglycerides as well as mixtures and combinations thereof, in particular wherein the triglycerides of Medium Chain Triglycerides (MCT) are preferably selected from triglycerides of C₆-C₁₂ fatty acid residues and/or in particular wherein the total amount of a further component or ingredient is 0.01 to 35 wt.-%, in particular 0.1 to 30 wt.-%, preferably 1 to 25 wt.-%, with respect to the second capsule shell amount; and/or
wherein the second capsule shell is made alkaline.

6. Dosage form according to one of the preceding claims,
wherein the dosage form containing the second capsule shell in amounts of 0.5 to 100 mg, in particular 1 to 75 mg, preferably 1 to 50 mg, more preferably 2 to 40 mg, particularly preferably 3 to 35 mg, very particularly preferably 5 to 30 mg, and/or wherein the dosage form has a weight-based proportion of the second capsule shell, depending on the dosage form, in the range of 2 to 4 wt.-%; and/or
wherein the diameter, in particular the average diameter, of the dosage form varies in the range 50 µm to 25 mm, in particular in the range of 100 µm to 25 mm, preferably in the range of 250 µm to 25 mm, more preferably in the range of 500 µm to 25 mm, very particularly preferably in the range of 750 µm to 25 mm, more preferably in the range of 1,000 µm to 25 mm; and/or
wherein the ratio of the thickness of the first capsule shell to the thickness of the second capsule shell is 500:1 to 1:1, especially 100:1 to 2:1, preferably 50:1 to 5:1; and/or
wherein the weight-ratio of the first to the second capsule shell capsule shell is 1:1 to 50:1, especially 2:1 to 30:1, preferably 3:1 to 25:1, more preferably 5:1 to 20:1.

7. Dosage form according to one of the preceding claims, wherein the dosage form is provided with an outer coating surrounding the second capsule shell, especially directly surrounding the second capsule shell, wherein it is based, in particular, on lacquers or waxes as well as mixtures and combinations thereof.

8. Dosage form according to one of the preceding claims
- for use in human medicine or veterinary medicine and/or for use in the prophylactic and/or therapeutic treatment of the human or animal body; or
- for use in the prophylactic and/or therapeutic treatment of inflammatory, infection-exacerbating or allergic diseases of the human or animal body; or
- for use in the prophylactic and/or therapeutic treatment of autoimmune diseases of the human or animal body; or
- for use in the prophylactic and/or therapeutic treatment of colds and flu and related diseases and infections, especially infections of the upper and lower respiratory tract, in particular rhinitis, sinusitis and broncho-pulmonary diseases; or
- for use in the prophylactic and/or therapeutic treatment of respiratory diseases, in particular of broncho-pulmonary diseases, in particular bronchitis, bronchial asthma and chronic obstructive pulmonary disease (COPD); or
- for use in the prophylactic and/or therapeutic treatment of inflammatory diseases of the biliary tract, and in particular cholecystitis and cholangitis; or
- for use in the prophylactic and/or therapeutic treatment of inflammatory diseases of the urinary tract, glomerulonephritis, pyelonephritis, cystitis and urethritis; or
- for use in the prophylactic and/or therapeutic treatment of inflammatory diseases of the intestine, in particular Crohn's disease and ulcerative colitis; or
- for use in the prophylactic and/or therapeutic treatment of inflammatory or allergic skin diseases, especially eczema and dermatitis; or
- for use in the prophylactic and/or therapeutic treatment of rheumatoid diseases, especially rheumatoid arthritis and/or rheumatism and diseases of the rheumatic type; or
- for use in the prophylactic and/or therapeutic treatment of systemic corticosteroid-treated diseases, in particular diseases that are treated with systemically administered corticosteroids.

9. Dosage form according to claim 8 wherein the corticosteroid, in particular 1,8-cineol, is administered in daily doses of 50 to 3,000 mg/diem, in particular 100 to 2,000 mg/diem, preferably 200 to 1,000 mg/diem, and/or wherein the monoterpene-containing active substance, in particular 1,8-cineol, is administered in daily doses of 50 to 3,000 mg/diem, in particular 100 to 2,000 mg/diem, preferably 200 to 1,000 mg/diem.

10. Medicinal product or medicines comprising a monoterpene-containing, in particular cineol-containing, dosage form according to one of the preceding claims.

11. Method for preparing a monoterpene-containing dosage form for peroral administration in the form of a capsule, especially in the form of a capsule having a core/shell structure with multi-layered shell, such as described in one of the preceding claims, wherein
(i) in a first method step, a capsule core, comprising at least a monoterpene-containing active substance, in particular at least a monoterpene, is provided, with a first capsule shell surrounding the capsule core, in particular directly surrounding the capsule core, in order to structure and/or form it, and
(ii) in a subsequent second method step, the first capsule shell is provided with a second capsule shell surrounding the first capsule shell, in particular directly surrounding the first capsule shell, preferably in the form of a coating, in particular in the form of a gastro-resistant but thin enteric coating, wherein at least two components are used for the production of the second capsule shell, wherein a first component ("component 1") based on alginic acid or physiologically compatible salts or esters is formed, and a second component ("component 2") based on cellulose is formed, wherein the first and the second component for the production of the second capsule shell may be applied jointly in the form of an aqueous solution and/or dispersion, wherein the solution and/or dispersion is made alkaline, and wherein the dosage form obtained has a weight-based proportion of the second capsule shell, depending on the dosage form, in the range of 0.5 to 4 wt.-%.

12. Method according to claim 11,
wherein the solution and/or dispersion is made alkaline using aqueous ammonia solution; and/or
wherein the solution and/or dispersion is applied at temperatures in the range of 10 to 70°C, especially 15 to 60°C, preferably 20 to 50°C, more preferably 25 to 40°C;
wherein the solution and/or dispersion is applied by means of immersion and/or spraying, especially by means of spraying; and/or
wherein in a final step, (iii) at least one coating, in particular based on lacquers or waxes as well as mixtures and combinations thereof, is applied to the second capsule shell, in particular, is directly applied to the second capsule shell.

## Revendications

1. Forme pharmaceutique contenant des monoterpènes, pour administration orale sous forme d'une capsule, en particulier sous forme d'une capsule ayant une structure noyau/enveloppe avec une enveloppe multicouche, la capsule présentant, de l'intérieur vers l'extérieur, la structure suivante :
(a) un noyau de capsule, contenant au moins un principe actif contenant des monoterpènes, en particulier au moins un monoterpène,
(b) une première enveloppe de capsule, entourant le noyau de capsule, en particulier entourant directement le noyau de capsule, définissant la structure et/ou la forme, et
(c) une deuxième enveloppe de capsule, entourant la première enveloppe de capsule, en particulier entourant directement la première enveloppe de capsule, sous forme d'un revêtement, en particulier sous forme d'un revêtement gastro-résistant mais soluble dans l'intestin grêle, la deuxième enveloppe de capsule comprenant au moins deux composants (ingrédients ou constituants), un premier composant ("composant 1") étant constitué à base d'acide alginique ou de ses sels ou esters physiologiquement compatibles, et un deuxième composant ("composant 2") à base de cellulose, la forme pharmaceutique présentant une proportion pondérale de la deuxième enveloppe de capsule, rapportée à la forme pharmaceutique, comprise dans la plage de 0,5 à 4 % en poids.

2. Forme pharmaceutique selon la revendication 1,
dans laquelle le principe actif contenant des monoterpènes, en particulier le monoterpène, est choisi dans le groupe consistant en le 1,8-cinéol, le menthol, le camphre, le limonène, la carvone, le carvéol ou les combinaisons de ceux-ci, en particulier dans lequel le monoterpène est le 1,8-cinéol et/ou le menthol, d'une manière particulièrement préférée le 1,8-cinéol ; et/ou
dans laquelle la forme pharmaceutique, en particulier le noyau de capsule, contient du 1,8-cinéol en tant que principe actif unique, ou encore dans laquelle la forme pharmaceutique, en particulier le noyau de capsule, contient du 1,8-cinéol avec au moins un autre principe actif, de préférence choisi parmi les terpènes.

3. Forme pharmaceutique selon l'une des revendications précédentes, **caractérisée en ce que** la forme pharmaceutique, en particulier le noyau de capsule, contient le principe actif contenant des monoterpènes et/ou le monoterpène, en particulier le 1,8-cinéol, en même temps qu'au moins un support (excipient), miscible au principe actif contenant des monoterpènes et/ou au monoterpène, en particulier au 1,8-cinéol, et/ou soluble dans ce dernier, liquide en particulier à 20°C et sous la pression atmosphérique, et physiologiquement inoffensif ;
en particulier dans laquelle le support est choisi dans le groupe consistant en les huiles grasses, en particulier les triglycérides, d'une manière particulièrement préférée les triglycérides à chaîne moyenne (*Medium Chain Triglycerides,* MCT), d'une manière tout particulièrement préférée les triglycérides ayant des résidus acides gras en C₆-C₁₂ ; et/ou
en particulier dans laquelle le support est utilisé selon un rapport en poids entre le principe actif monoterpénique et le support compris dans la plage de 1000:1 à 1:1000, en particulier de 100:1 à 1:100, de préférence de 50:1 à 1:50, d'une manière particulièrement préférée de 10:1 à 1:10, d'une manière tout particulièrement préférée de 5:1 à 1:2, d'une manière encore plus préférée de 3:1 à 1:1.

4. Forme pharmaceutique selon l'une des revendications précédentes,
dans laquelle la forme pharmaceutique contient le principe actif contenant des monoterpènes en des quantités absolues de 10 à 1000 mg, en particulier de 25 à 750 mg, de préférence de 50 à 500 mg, d'une manière particulièrement préférée de 75 à 300 mg, en particulier rapportées à une unité posologique, en particulier à une capsule ; et/ou
dans laquelle la forme pharmaceutique contient le principe actif contenant des monoterpènes en des quantités relatives de 0,01 à 99 % en poids, en particulier de 0,1 à 95 % en poids, de préférence de 1 à 90 % en poids, d'une manière particulièrement préférée de 5 à 80 % en poids, rapportées à la forme posologique ; et/ou
dans laquelle la forme pharmaceutique contient le noyau de capsule en des quantités de 10 à 1500 mg, en particulier de 50 à 1250 mg, de préférence de 75 à 1000 mg, préférentiellement de 100 à 750 mg, d'une manière particulièrement préférée de 125 à 500 mg, et/ou dans laquelle la forme pharmaceutique présente une proportion pondérale du noyau de capsule, rapportée à la forme posologique, comprise dans la plage de 10 à 90 % en poids, en particulier de 20 à 85 % en poids, de préférence de 40 à 80 % en poids, préférentiellement de 50 à 75 % en poids, d'une manière particulièrement préférée de 60 à 70 % en poids ; et/ou
dans laquelle la première enveloppe de capsule est conçue comme une capsule molle et/ou une enveloppe de capsule molle, et/ou dans laquelle le matériau de l'enveloppe de capsule de la première enveloppe de capsule comprend au moins un polymère pharmacologiquement inoffensif ou en est constitué, en particulier choisi parmi les polymères et produits de polymérisation synthétiques, naturels ou identiques au naturel, de préférence choisis dans le groupe des protéines, de préférence des gélatines et/ou des caséinates, de préférence des gélatines, ainsi que des mélanges et combinaisons de ceux-ci ; et/ou
dans laquelle le matériau d'enveloppe de capsule de la première enveloppe de capsule comprend de la gélatine, en particulier de la gélatine dure ou de la gélatine molle, de préférence de la gélatine molle, ou en est constitué, éventuellement en même temps qu'un autre additif ou adjuvant, choisi en particulier parmi les plastifiants, les humectants et les lubrifiants, ainsi que les mélanges et combinaisons de ceux-ci, en particulier dans laquelle la quantité d'additif ou d'adjuvant est en tout de 0,01 à 40 % en poids, en particulier de 0,1 à 35 % en poids, de préférence de 1 à 30 % en poids, par rapport à la première enveloppe de capsule, et/ou
dans laquelle la forme pharmaceutique contient la première enveloppe de capsule en des quantités de 40 à 500 mg, en particulier de 50 à 400 mg, de préférence de 60 à 300 mg, de préférence de 70 à 250 mg, d'une manière particulièrement préférée de 80 à 200 mg, et/ou dans laquelle la forme pharmaceutique présente une proportion pondérale de la première enveloppe de capsule, rapportée à la forme pharmaceutique, comprise dans la plage de 15 à 75 % en poids, en particulier de 20 à 65 % en poids, de préférence de 25 à 55 % en poids, de préférence de 30 à 50 % en poids, d'une manière particulièrement préférée de 35 à 45 % en poids.

5. Forme pharmaceutique selon l'une des revendications précédentes,
dans laquelle la deuxième enveloppe de capsule présente en tant que premier composant au moins un alginate, en particulier choisi parmi les alginates de métaux alcalins, les alginates de métaux alcalino-terreux, les alginates d'ammonium et les esters de l'acide alginique, de préférence choisis parmi l'alginate de sodium, l'alginate de potassium, l'alginate d'ammonium, l'alginate de calcium et l'alginate de propylèneglycol, choisi de préférence parmi l'alginate de sodium, l'alginate de potassium, l'alginate d'ammonium et l'alginate de calcium, d'une manière particulièrement préférée l'alginate de sodium, et/ou
dans laquelle la deuxième enveloppe de capsule contient en tant que deuxième composant de la cellulose ou un dérivé de la cellulose, en particulier un éther de cellulose ou un ester de cellulose, en particulier dans laquelle le deuxième composant est choisi parmi les éthers de cellulose, de préférence du groupe consistant en la méthylcellulose, l'éthylcellulose, l'éthylméthylcellulose, l'hydroxypropylcellulose, l'hydroxypropylméthylcellulose, la carboxyméthylcellulose ou les combinaisons de celles-ci, de préférence l'éthylcellulose, et/ou
dans laquelle la deuxième enveloppe de capsule contient le premier et le deuxième composants selon des proportions pondérales du premier composant au deuxième composant ("rapport en poids composant 1/composant 2") de 1:99 à 50:50, en particulier de 5:95 à 40:60, de préférence de 8:92 à 30:70, préférentiellement de 10:90 à 25:75, d'une manière particulièrement préférée de 12:88 à 20:80 ; et/ou
dans laquelle la deuxième enveloppe de capsule comprend en outre au moins un autre constituant ou ingrédient, en particulier choisi parmi les additifs ou les adjuvants, de préférence parmi les huiles grasses et/ou les acides gras, d'une manière particulièrement préférée choisis dans le groupe consistant en l'acide stéarique, l'acide oléique et les triglycérides, ainsi que les mélanges et combinaisons de ceux-ci, en particulier dans laquelle les triglycérides sont choisis parmi les triglycérides à chaîne moyenne (*Medium Chain Triglycerides,* MCT), de préférence les triglycérides avec des esters d'acides gras en C₆-C₁₂, et/ou en particulier dans laquelle la quantité de l'autre constituant ou ingrédient peut en tout être de 0,01 à 35 % en poids, en particulier de 0,1 à 30 % en poids, de préférence de 1 à 25 % en poids, par rapport à la deuxième enveloppe de capsule ; et/ou
dans laquelle la deuxième enveloppe de capsule est ajustée à des conditions basiques.

6. Forme pharmaceutique selon l'une des revendications précédentes,
dans laquelle la forme pharmaceutique contient la deuxième enveloppe de capsule en des quantités de 0,5 à 100 mg, en particulier de 1 à 75 mg, de préférence de 1 à 50 mg, préférentiellement de 2 à 40 mg, d'une manière particulièrement préférée de 3 à 35 mg, d'une manière tout particulièrement préférée de 5 à 30 mg, et/ou la forme pharmaceutique présente une proportion en poids de la deuxième enveloppe de capsule, rapportée à la forme pharmaceutique, comprise dans la plage de 2 à 4 % en poids ; et/ou
dans laquelle le diamètre, en particulier le diamètre moyen, de la forme pharmaceutique varie dans la plage de 50 µm à 25 mm, en particulier dans la plage de 100 µm à 25 mm, de préférence dans la plage de 250 µm à 25 mm, d'une manière particulièrement préférée dans la plage de 500 µm à 25 mm, d'une manière tout particulièrement préférée dans la plage de 750 µm à 25 mm, d'une manière encore plus préférée dans la plage de 1000 µm à 25 mm ; et/ou
dans laquelle le rapport de l'épaisseur de la première enveloppe de capsule à l'épaisseur de la deuxième enveloppe de capsule est de 500:1 à 1:1, en particulier de 100:1 à 2:1, de préférence de 50:1 à 5:1 ; et/ou
dans laquelle le rapport en poids de la première enveloppe de capsule à la deuxième enveloppe de capsule est de 1:1 à 50:1, en particulier de 2:1 à 30:1, de préférence de 3:1 à 25:1, préférentiellement de 5:1 à 20:1.

7. Forme pharmaceutique selon l'une des revendications précédentes, dans laquelle la forme pharmaceutique est pourvue d'un revêtement extérieur, entourant la deuxième enveloppe de capsule, en particulier entourant directement la deuxième enveloppe de capsule, en particulier à base de vernis ou de cires, ainsi que de mélanges et combinaisons de ceux-ci.

8. Forme pharmaceutique selon l'une des revendications précédentes,
pour une utilisation en médecine humaine ou en médecine vétérinaire et/ou pour une utilisation lors du traitement prophylactique et/ou thérapeutique du corps humain ou animal ; ou
pour une utilisation lors du traitement prophylactique et/ou thérapeutique de maladies inflammatoires, exacerbées par une infection ou allergiques, du corps humain ou animal ; ou
pour une utilisation lors du traitement prophylactique et/ou thérapeutique de maladies auto-immunes du corps humain ou animal ; ou
pour une utilisation lors du traitement prophylactique et/ou thérapeutique de maladies de refroidissement et d'infections grippales et de maladies et infections qui leur sont associées, en particulier les infections des voies respiratoires supérieures et inférieures, en particulier la rhinite, la sinusite et les maladies broncho-pulmonaires ; ou
pour une utilisation lors du traitement prophylactique et/ou thérapeutique des maladies des voies respiratoires, en particulier des maladies broncho-pulmonaires, en particulier la bronchite, l'asthme bronchique et les broncho-pneumopathies chroniques obstructives (BPCO) ; ou
pour une utilisation lors du traitement prophylactique et/ou thérapeutique de maladies inflammatoires des voies biliaires, en particulier la cholécystite et la cholangite ; ou
pour une utilisation lors du traitement prophylactique et/ou thérapeutique de maladies inflammatoires des voies urinaires excrétrices, en particulier la glomérulonéphrite, la pyélonéphrite, la cystite et l'urétrite ; ou
pour une utilisation lors du traitement prophylactique et thérapeutique de maladies inflammatoires de l'intestin, en particulier la maladie de Crohn et la recto-colite hémorragique ; ou
pour une utilisation lors du traitement prophylactique et/ou thérapeutique de maladies inflammatoires ou allergiques de la peau, en particulier les eczémas et la dermatite ; ou
pour une utilisation lors du traitement prophylactique et/ou thérapeutique de maladies rhumatoïdes, en particulier le rhumatisme et/ou les maladies rhumatismales, ainsi que les maladies présentant des manifestations rhumatismales ; ou
pour une utilisation lors du traitement prophylactique et/ou thérapeutique de maladies exigeant un traitement aux corticostéroïdes systémiques, en particulier les maladies qui sont traitées par des corticostéroïdes administrés par voie systémique.

9. Forme pharmaceutique selon la revendication 8, dans laquelle le principe actif contenant des monoterpènes, en particulier du 1,8-cinéol, est administré à des doses journalières de 50 à 3000 mg/jour, en particulier de 100 à 2000 mg/jour, de préférence de 200 à 1000 mg/jour, et/ou dans laquelle le principe actif contenant des monoterpènes, en particulier du 1,8-cinéol, est conçu pour administration à une dose journalière de 50 à 3000 mg/jour, en particulier de 100 à 2000 mg/jour, de préférence de 200 à 1000 mg/jour.

10. Médicament ou produit médical, comprenant une forme pharmaceutique contenant des monoterpènes, en particulier contenant du cinéol, selon l'une des revendications précédentes.

11. Procédé de fabrication d'une forme pharmaceutique contenant des monoterpènes pour administration par voie orale sous forme d'une capsule, en particulier sous forme d'une capsule à structure noyau/enveloppe avec une enveloppe multicouche, telle que définie dans l'une des revendications précédentes, dans lequel
(i) dans une première étape du procédé, on pourvoit un noyau de capsule, contenant au moins un principe actif contenant des monoterpènes, en particulier au moins un monoterpène, d'une première enveloppe de capsule, entourant le noyau de capsule, en particulier entourant directement le noyau de capsule, assurant la structure et/ou la forme, et
(ii) dans une deuxième étape du procédé, mise en oeuvre ensuite, on pourvoit la première enveloppe de capsule d'une deuxième enveloppe de capsule, entourant la première enveloppe de capsule, en particulier entourant directement la première enveloppe de capsule, de préférence sous forme d'un revêtement, en particulier sous forme d'un revêtement gastro-résistant mais soluble dans l'intestin grêle, auquel cas, pour fabriquer la deuxième enveloppe de capsule, on utilise au moins deux composants, un premier composant ("composant 1") étant constitué à base d'acide alginique ou de ses sels ou esters physiologiquement compatibles, et un deuxième composant ("composant 2") à base de cellulose, le premier et le deuxième composants étant, pour la fabrication de la deuxième enveloppe de capsule, appliqués ensemble sous forme d'une solution et/ou d'une dispersion aqueuse, la solution et/ou la dispersion étant ajustées à des conditions basiques, et la forme pharmaceutique obtenue présentant une proportion pondérale de la deuxième enveloppe de capsule, rapportée à la forme pharmaceutique, comprise dans la plage de 0,5 à 4 % en poids.

12. Procédé selon la revendication 11,
dans lequel la solution et/ou la dispersion sont ajustées à des conditions basiques à l'aide d'une solution d'ammoniaque ; et/ou
dans lequel la solution et/ou la dispersion sont appliquées à des températures comprises dans la plage de 10 à 70°C, en particulier de 15 à 60°C, de préférence de 20 à 50°C, préférentiellement de 25 à 40°C ;
dans lequel la solution et/ou la dispersion sont appliquées par des procédés au trempé et/ou par pulvérisation, en particulier par des procédés par pulvérisation ; et/ou
dans lequel, dans une étape finale (iii), au moins un revêtement, en particulier à base de vernis ou de cires ainsi que des mélanges et combinaisons de ceux-ci, est appliqué sur la deuxième enveloppe de capsule, en particulier est appliqué directement sur la deuxième enveloppe de capsule.
